# EUROPEAN PATENT APPLICATION

(11) **EP 4 039 799 A1**
(43) Date of publication of application: **10.08.2022**
(21) Application number: 20905498.0
(22) Date of filing: 28.09.2020
(51) Int. Cl.: C12N 5/0783, A61K 35/17

(54) **ENGINEERED IMMUNE KILLER CELL, PREPARATION METHOD THEREFOR AND USE THEREOF**

(30) Priority: 27.12.2019 CN 201911382947
(71) Applicant: Zhaotai Immugene Biomedicine (Hong Kong) Limited, Hong Kong 999077 (HK)
(72) Inventor: LI, Peng, Hong Kong 999077 (CN); JIANG, Zhiwu, Hong Kong 999077 (CN); TANG, Zhaoyang, Hong Kong 999077 (CN); QIN, Le, Hong Kong 999077 (CN); LIAO, Rui, Hong Kong 999077 (CN); ZHENG, Diwei, Hong Kong 999077 (CN); CUI, Yuanbin, Hong Kong 999077 (CN); YAO, Yao, Hong Kong 999077 (CN); LIN, Simiao, Hong Kong 999077 (CN)
(74) Representative: Freyria Fava, Cristina
(86) International application number: PCT/CN2020/118265
(87) International publication number: WO 2021/129015

(57) **Abstract**

Disclosed are an engineered immune killer cell, and a preparation method therefor and the use thereof. The engineered immune killer cell is prepared by inducing reprogrammed human T cell, retains the marker and function of the human T cell from which the engineered immune killer cell is derived, has the marker and function of an NK cell, and transfects and expresses, in an obtained immune killer lymphocyte, a CAR molecule which recognizes tumor and virus-associated antigens or a TCR molecule which specifically recognizes a tumor.

SEQUENCE LISTING

<110>  ZHAOTAI IMMUGENE BIOMEDICINE (HONG KONG) LIMITED

<120>  ENGINEERED IMMUNE KILLER CELL, PREPARATION METHOD THEREFOR AND
       USE THEREOF

<130>  BY20DX0932FPPC-CN

<160>  51

<170>  PatentIn version 3.3

<210>  1
<211>  20
<212>  DNA
<213>  Homo sapiens

<400>  1
gaccctgacc tgctcacctg                                                   20


<210>  2
<211>  24
<212>  DNA
<213>  Artificial sequence

<220>
<223>  Forward primer for the second exon target sequence

<400>  2
caccgaccct gacctgctca cctg                                              24


<210>  3
<211>  24
<212>  DNA
<213>  Artificial sequence

<220>
<223>  Reverse primer for the second exon target sequence

<400>  3
aaaccaggtg agcaggtcag ggtc                                              24


<210>  4
<211>  20
<212>  DNA
<213>  Homo sapiens

<400>  4
gaagcagtgt ggcggcagct                                                   20


<210>  5
<211>  24
<212>  DNA
<213>  Artificial sequence

<220>
<223>  Forward primer for the second exon target sequence

<400>  5
caccgaagca gtgtggcggc agct                                              24


<210>  6
<211>  24
<212>  DNA
<213>  Artificial sequence

<220>
<223>  Reverse primer for the second exon target sequence

<400>  6
aaacagctgc cgccacactg cttc                                              24


<210>  7
<211>  20
<212>  DNA
<213>  Homo sapiens

<400>  7
caggtggtca tcttcgtcgg                                                   20


<210>  8
<211>  24
<212>  DNA
<213>  Artificial sequence

<220>
<223>  Forward primer for the second exon target sequence

<400>  8
cacccaggtg gtcatcttcg tcgg                                              24


<210>  9
<211>  24
<212>  DNA
<213>  Artificial sequence

<220>
<223>  Reverse primer for the second exon target sequence

<400>  9
aaacccgacg aagatgacca cctg                                              24


<210>  10
<211>  19
<212>  DNA
<213>  Homo sapiens

<400>  10
gcaggtggtc atcttcgtc                                                    19


<210>  11
<211>  23
<212>  DNA
<213>  Artificial sequence

<220>
<223>  Forward primer for the second exon target sequence

<400>  11
caccgcaggt ggtcatcttc gtc                                              23


<210>  12
<211>  23
<212>  DNA
<213>  Artificial sequence

<220>
<223>  Reverse primer for the second exon target sequence

<400>  12
aaaccgacga agatgaccac ctg                                              23


<210>  13
<211>  20
<212>  DNA
<213>  Homo sapiens

<400>  13
gctcaggaaa gtgtccgagc                                                  20


<210>  14
<211>  24
<212>  DNA
<213>  Artificial sequence

<220>
<223>  Forward primer for the second exon target sequence

<400>  14
caccgctcag gaaagtgtcc gagc                                             24


<210>  15
<211>  23
<212>  DNA
<213>  Artificial sequence

<220>
<223>  Reverse primer for the second exon target sequence

<400>  15
aaacgctcgg acactttcct gag                                              23


<210>  16
<211>  20
<212>  DNA
<213>  Homo sapiens

<400>  16
gagtcccgtc acccgagacc                                                  20


<210>  17
<211>  24

<212>  DNA
<213>  Artificial sequence

<220>
<223>  Forward primer for the third exon target sequence

<400>  17
caccgagtcc cgtcacccga gacc                                              24


<210>  18
<211>  23
<212>  DNA
<213>  Artificial sequence

<220>
<223>  Reverse primer for the third exon target sequence

<400>  18
aaacggtctc gggtgacggg act                                               23


<210>  19
<211>  20
<212>  DNA
<213>  Homo sapiens

<400>  19
gaagtgatca cggatgagtg                                                   20


<210>  20
<211>  24
<212>  DNA
<213>  Artificial sequence

<220>
<223>  Forward primer for the third exon target sequence

<400>  20
caccgaagtg atcacggatg agtg                                              24


<210>  21
<211>  23
<212>  DNA
<213>  Artificial sequence

<220>
<223>  Reverse primer for the third exon target sequence

<400>  21
aaaccactca tccgtgatca ctt                                               23


<210>  22
<211>  20
<212>  DNA
<213>  Homo sapiens

<400>  22
ggtgacggga ctcagggtga                                                   20

<210>  23
<211>  24
<212>  DNA
<213>  Artificial sequence

<220>
<223>  Forward primer for the third exon target sequence

<400>  23
caccggtgac gggactcagg gtga                                              24


<210>  24
<211>  23
<212>  DNA
<213>  Artificial sequence

<220>
<223>  Reverse primer for the third exon target sequence

<400>  24
aaactcaccc tgagtcccgt cac                                               23


<210>  25
<211>  20
<212>  DNA
<213>  Homo sapiens

<400>  25
tgcagcgcgc gcccggtctc                                                   20


<210>  26
<211>  24
<212>  DNA
<213>  Artificial sequence

<220>
<223>  Forward primer for the third exon target sequence

<400>  26
cacctgcagc gcgcgcccgg tctc                                              24


<210>  27
<211>  24
<212>  DNA
<213>  Artificial sequence

<220>
<223>  Reverse primer for the third exon target sequence

<400>  27
aaacgagacc gggcgcgcgc tgca                                              24


<210>  28
<211>  20
<212>  DNA
<213>  Homo sapiens

<400>  28
cacgagagcg acccgtcgct                                                   20


<210>  29
<211>  24
<212>  DNA
<213>  Artificial sequence

<220>
<223> Forward primer for the fourth exon target sequence

<400>  29
cacccacgag agcgacccgt cgct                                              24


<210>  30
<211>  24
<212>  DNA
<213>  Artificial sequence

<220>
<223>  Reverse primer for the fourth exon target sequence

<400>  30
aaacagcgac gggtcgctct cgtg                                              24


<210>  31
<211>  20
<212>  DNA
<213>  Homo sapiens

<400>  31
gcgacgggtc gctctcgtgg                                                   20


<210>  32
<211>  24
<212>  DNA
<213>  Artificial sequence

<220>
<223> Forward primer for the fourth exon target sequence

<400>  32
caccgcgacg ggtcgctctc gtgg                                              24


<210>  33
<211>  23
<212>  DNA
<213>  Artificial sequence

<220>
<223>  Reverse primer for the fourth exon target sequence

<400>  33
aaacccacga gagcgacccg tcg                                               23


<210>  34
<211>  20

<212>  DNA
<213>  Homo sapiens

<400>  34
tccatgctga agctcgactc                                                   20


<210>  35
<211>  24
<212>  DNA
<213>  Artificial sequence

<220>
<223> Forward primer for the fourth exon target sequence

<400>  35
cacctccatg ctgaagctcg actc                                              24


<210>  36
<211>  24
<212>  DNA
<213>  Artificial sequence

<220>
<223>  Reverse primer for the fourth exon target sequence

<400>  36
aaacgagtcg agcttcagca tgga                                              24


<210>  37
<211>  20
<212>  DNA
<213>  Homo sapiens

<400>  37
acgggtcgct ctcgtggtgg                                                   20


<210>  38
<211>  24
<212>  DNA
<213>  Artificial sequence

<220>
<223> Forward primer for the fourth exon target sequence

<400>  38
caccacgggt cgctctcgtg gtgg                                              24


<210>  39
<211>  24
<212>  DNA
<213>  Artificial sequence

<220>
<223>  Reverse primer for the fourth exon target sequence

<400>  39
aaacccacca cgagagcgac ccgt                                              24

<210>  40
<211>  20
<212>  DNA
<213>  Homo sapiens

<400>  40
agccgcaacc gcgagaacgg                                                   20


<210>  41
<211>  24
<212>  DNA
<213>  Artificial sequence

<220>
<223> Forward primer for the fourth exon target sequence

<400>  41
caccagccgc aaccgcgaga acgg                                              24


<210>  42
<211>  24
<212>  DNA
<213>  Artificial sequence

<220>
<223>  Reverse primer for the fourth exon target sequence

<400>  42
aaacccgttc tcgcggttgc ggct                                              24


<210>  43
<211>  20
<212>  DNA
<213>  Homo sapiens

<400>  43
gcaacttgac ggtgcaccgg                                                   20


<210>  44
<211>  24
<212>  DNA
<213>  Artificial sequence

<220>
<223> Forward primer for the fourth exon target sequence

<400>  44
caccgcaact tgacggtgca ccgg                                              24


<210>  45
<211>  23
<212>  DNA
<213>  Artificial sequence

<220>
<223>  Reverse primer for the fourth exon target sequence

<400>  45
aaacccggtg caccgtcaag ttg                                              23


<210>  46
<211>  20
<212>  DNA
<213>  Homo sapiens

<400>  46
gagctgggcc gcccggggcc                                                  20


<210>  47
<211>  24
<212>  DNA
<213>  Artificial sequence

<220>
<223> Forward primer for the fourth exon target sequence

<400>  47
caccgagctg ggccgcccgg ggcc                                             24


<210>  48
<211>  23
<212>  DNA
<213>  Artificial sequence

<220>
<223>  Reverse primer for the fourth exon target sequence

<400>  48
aaacggcccc gggcggccca gct                                              23


<210>  49
<211>  20
<212>  DNA
<213>  Homo sapiens

<400>  49
ggtcagacgg aggctccctt                                                  20


<210>  50
<211>  24
<212>  DNA
<213>  Artificial sequence

<220>
<223>  Forward primer for the third exon target sequence

<400>  50
caccggtcag acggaggctc cctt                                             24


<210>  51
<211>  24
<212>  DNA
<213>  Artificial sequence

<220>
<223>  Reverse primer for the third exon target sequence

<400>  51
aaacaaggga gcctccgtct gacc                                              24

## Description

### TECHNICAL FIELD

The present application relates to the technical field of biomedicine and, in particular, to an engineered immune killer cell, a preparation method therefor, and a use thereof.

### BACKGROUND

At present, immunotherapy has become the most concerned and promising "new" idea in the field of cancer treatment. Among the top 10 scientific breakthroughs of the year ranked by *Science* magazine in 2013, tumor immunotherapy tops the list. Chimeric antigen receptor (CAR) T cells of Novartis and Kite have been approved by the U.S. FDA, and tumor immune cell therapy has made milestone progress in the treatment field of hematological malignancies. However, tumor immune cell therapy still has technical bottlenecks in the clinical treatment. For example, genetically modified immune cells have a single target, causing tumor immune escape and tumor recurrence; a solid tumor lacks a specific marker with high efficiency and low side effects, and current genetically modified immune cell therapy has no effective and safe clinical effect on the solid tumor.

T cells can be divided into different subgroups according to their surface markers and functions. For example, T cells can be divided into γδT cells and αβT cells according to the types of TCRs. αβT cells, which account for more than 95% of T cells, are the main cell population having T cell differentiation markers *in vivo* and performing T cell functions and represent the diversity of T cells. γδT cells are a group of highly heterogeneous cells. A T-cell receptor on the surface of γδT cells is composed of a γ chain and a δ chain. γδT cells have many subtypes, variable phenotypes, and rich functions. The subtypes of γδT cells have different biological characteristics, and γδT cells play an important role in the occurrence and development of tumors, infections, and autoimmune diseases in the body and are considered to be a bridge of the body which links innate immunity to adaptive immunity.

Like T cells, natural killer (NK) cells are an indispensable part of a human immune system. NK cells are considered to be lymphoid cells which account for about 10% to 15% of peripheral blood lymphocytes and play a key role in an innate immune response. Different from T cells, NK cells recognize their targets in an MHC-unrestricted manner. NK cells have antiviral, anti-GvH, and anti-cancer effects. Specifically, NK cells directly kill malignant tumors including sarcomas, myelomas, cancers, lymphomas, and leukemia or eliminate abnormal cells by inducing the activity of dendritic cells (DCs) or the adaptive immune activation of tumor-specific cytotoxic T lymphocytes (CTLs), where the abnormal cells are tumor cells or cells that are developing into tumor cells. Although NK cells have the potential to be used as a therapeutic agent against cancer or infectious diseases, most NK cells in a normal human body are present in a dormant state, and NK cells in cancer patients lose their functions due to the immune escape mechanism of cancer cells. To use natural killer cells as the therapeutic agent in practice, activated natural killer cells that can recognize and destroy tumor cells are required. Since the number of natural killer cells in the body is limited, it is very important to obtain a sufficient number of activated natural killer cells.

Cell reprogramming refers to the process in which differentiated cells are reversed under particular conditions and then return to a totipotent state or form an embryonic stem cell line or further develop into a new individual. In the field of immunotherapy of diseases, there have been reports on the transformation of immune cells through cell reprogramming. For example, Dr. Ding Sheng from Gladstone Institutes in the U.S. has reported that pro-inflammatory effector T cells are reprogrammed into anti-inflammatory regulatory T cells by a particular reprogramming method. Such reprogramming is of great significance to the treatment of autoimmune diseases. Specifically, for autoimmune diseases, over-stimulated effector T cells cause damages to the body, and when these cells are transformed into regulatory T cells, the overactivity of the immune system can be reduced and thus the immune system restores its balance, thereby fundamentally treating the diseases. At present, cell reprogramming is still to be applied to human tumor immunotherapy.

A chimeric antigen receptor (CAR) molecule typically includes an extracellular fragment, a transmembrane region, and an intracellular fragment. The extracellular fragment is a single-chain variable fragment (scFv) formed by linking heavy and light chain variable regions of an antibody via a peptide fragment. The intracellular fragment is a chimera of various signal transduction molecules including CD3zeta, CD28, OX-40, 4-1BB, and the like. The transmembrane region is derived from the transmembrane region of another molecule (such as CD8, CD4, CD28, and CD3zeta). The genes of the single-chain variable fragment are isolated from, for example, hybridomas that produce monoclonal antibodies that recognize target antigens.

The structural design of the CAR molecule has undergone many generations of research and development. The structure of the first-generation CAR molecule includes a single-chain variable fragment (scFv) that recognizes a tumor cell surface antigen, a transmembrane domain, and an intracellular domain that activates the TCR complex CD3ζ of T cells. Since the intracellular fragment of the first-generation CAR includes only a CD3ζ signal transduction region and no costimulatory signal, the first-generation CAR-T cells have severely deficient functions and show low levels in terms of the proliferation, persistence and effector functions in patients. For the purpose of enhancing the ability of the first-generation CAR to activate T cells, the second-generation CAR has been developed. The second-generation CAR adds an intracellular signal transduction domain derived from costimulatory molecules (such as CD28, CD134(OX-40), and CD137(4-1BB)). Clinical trials show that the second-generation CAR-T cells have relatively good proliferation, persistence, and effector functions in patients. The clinical trials of the second-generation CAR-T cells are mostly the treatment of B-cell leukemia with anti-CD 19 CAR-T cells. Although CAR-T cells have achieved efficacy in clinical trials, the CAR-T cells are to be further improved. The third-generation CAR is developed to further improve the efficacy of CAR-T cell therapy. The signal transduction regions of two costimulatory molecules are introduced into the intracellular fragment of the third-generation CAR. Typically, one costimulatory signal is the intracellular region of CD28, and the other costimulatory signal is the intracellular signal transduction region of CD134, CD137, ICOS or the like. Different combinations of costimulatory signals may affect the function and efficacy of CAR-T cells. Studies show that not all third-generation CARs are better than second-generation CARs.

Immune cells expressing CAR molecules can have an important anti-tumor effect. For example, CAR-T cells are independent of the expression of major histocompatibility antigens of type I on tumor cells, directly recognize tumor cell surface antigens, and simultaneously activate T cells so that the T cells expressing the CAR can effectively kill tumor cells. In short, CAR-T cells recognize specific molecules on the surface of tumor cells through an antigen-antibody recognition pattern and then are activated, proliferate, and kill cells through intracellular signaling.

### SUMMARY

In view of the defects in the existing art and practical requirements, the present application provides an engineered immune killer cell, a preparation method therefor, and a use thereof. The engineered immune killer cell of the present application has some markers and functions of both T cells and NK cells and simultaneously expresses antigen recognition and killing receptors of NK cells and T cells so that the engineered immune killer cell has more extensive tumor antigen recognition and killing functions than NK cells and T cells. Compared with a mature human T cell from which the engineered human immune cell is derived, the engineered human immune cell of the present application has an enhanced proliferation ability and a better anti-tumor effect. At the same time, the engineered human immune cell also has enhanced tumor-specific recognition and killing functions due to its CAR molecule expressing a tumor-associated antigen or its tumor-specific TCR molecule.

In a first aspect, the present application provides an engineered immune killer cell (hereinafter referred to as a CAR ITNK cell) prepared by transfecting a human T cell with a CAR molecule or a TCR molecule targeting a tumor-associated antigen or a virus-associated antigen along with or followed by reprogramming involving deletion or inhibition of a BCL11B gene.

Preferably, the immune killer cell expresses the CAR molecule or the TCR molecule targeting the tumor-associated antigen or the virus-associated antigen, retains a marker and a function of the human T cell from which the immune killer cell is derived, and has a marker and a function of NK cells.

Preferably, the human T cell is a mature human T cell or a cell population containing mature human T cells. Further preferably, the mature human T cell or the cell population containing mature human T cells is derived from cord blood or peripheral blood of a human body. Further preferably, the mature human T cell or the cell population containing mature human T cells is derived from a mature T cell or a cell population obtained through differentiation of pluripotent stem cells, embryonic stem cells, or cord blood stem cells.

Preferably, the reprogrammed immune killer lymphocyte expresses functional TCR, CD3, and NKp30.

Preferably, the reprogrammed immune killer lymphocyte expresses the following marker of NK cells: CD11c, NKG2D, and CD161.

Preferably, the reprogrammed immune killer lymphocyte performs low expression or no expression of an immunosuppression checkpoint PD-1, CTLA-4, or FOXP3.

Preferably, the reprogrammed immune killer lymphocyte performs low expression or no expression of an NK-associated marker CD127, CD16, KIRDL2, KIRDL3, NKG2A.

Preferably, the reprogrammed immune killer lymphocyte upregulates expression of NOTCH compared with the T cell from which the reprogrammed immune killer lymphocyte is derived.

Preferably, the reprogrammed immune killer lymphocyte downregulates expression of transcription factors LEF1 and TCF7 and upregulates expression of NOTCH, AP1, mTOR, ID2, TBX21, and NFIL3 compared with the T cell from which the reprogrammed immune killer lymphocyte is derived.

Preferably, TCR-mediated signal transduction of the reprogrammed immune killer lymphocyte is enhanced.

Preferably, compared with the T cell from which the reprogrammed immune killer lymphocyte is derived, the reprogrammed immune killer lymphocyte upregulates expression of genes CSF2, FOS, MAPK12, MAP3K8, IPNγ, NFKBIA, MAPK11, IL-10, and TEC which are associated with the TCR-mediated signal transduction.

Preferably, compared with NK cells, the reprogrammed immune killer lymphocyte has enhanced T cell recognition and TCR signal transduction; preferably, the reprogrammed immune killer lymphocyte upregulates expression of CD3, CD4, CD8, and CD40LG.

Preferably, compared with the T cell from which the reprogrammed immune killer lymphocyte is derived, the reprogrammed immune killer lymphocyte has enhanced NK killing toxicity-associated signal transduction.

Preferably, compared with the T cell from which the reprogrammed immune killer lymphocyte is derived, the reprogrammed immune killer lymphocyte upregulates expression of genes PRF1, CSF2, ICAM1, CD244, PLCG2, IFNG, FCER1G, GZMB, NCR2, NCR1, KIR2DL4, and SYK which are associated with the NK killing toxicity-associated signal transduction.

In a preferred specific embodiment, the reprogrammed immune killer lymphocyte includes CD8+NKp46^{hi}NKp44+NKp30+, CD4+NKp30+, and γδTCR+NKp46^{hi}NKp44+ NKp30+ T cell subgroups.

In a preferred specific embodiment, the human T cell is a mature human T cell, and reprogramming the mature human T cell includes:
(1') activating the mature human T cell;
(2') performing BCL11B gene knockout on the activated mature human T cell obtained in step (1'); and
(3') culturing the cell obtained in step (2') in a T cell culture medium.

In step (1'), the mature human T cell is activated using an anti-human CD3 antibody, an anti-human CD28 antibody, and an anti-human CD2 antibody.

Preferably, the T cell is activated through incubation of magnetic beads of the anti-human CD3 antibody, the anti-human CD28 antibody, and the anti-human CD2 antibody mixed with the mature human T cell at a ratio of 1:2.

In step (2'), the BCL11B gene knockout is performed using CRISPR/CAS9 technology.

Preferably, a target of the gene knockout is at a second exon of the BCL11B gene.

Preferably, the target of the gene knockout is at a third exon of the BCL11B gene.

In step (3'), the T cell culture medium includes IL-2; preferably, the cell obtained in step (2') is not co-cultured with OP9-DL1.

The CAR molecule includes the following domains: a signal peptide, an extracellular antigen recognition domain, a transmembrane region, and an intracellular costimulatory domain. In a preferred specific embodiment, the CAR molecule includes the signal peptide, the extracellular antigen recognition domain, the transmembrane region, and the intracellular costimulatory domain in sequence from an N-terminal to a C-terminal.

The tumor-associated antigen is a tumor surface antigen, a cytokine secreted by a tumor, a surface antigen of a cell associated with immunosuppression of a tumor microenvironment and a cytokine secreted by the cell, or a tumor-associated microbial antigen, preferably CD19, GPC3, Mesothelin, PSCA, or MUC1.

In a second aspect, the present application provides a method for preparing the cell according to the first aspect. The method includes:
(1") activating a human T cell;
(2") transfecting the activated human T cell with a CAR molecule expressing a tumor-associated antigen or a tumor-specific TCR molecule along with or followed by performing BCL11B gene knockout; and
(3") culturing the cell obtained in step (2") in a T cell culture medium.

In the preceding method, preferably, in step (1"), the human T cell is a mature human T cell or a cell population containing mature human T cells; further preferably, the mature human T cell or the cell population containing mature human T cells is derived from cord blood or peripheral blood of a human body; further preferably, the mature human T cell or the cell population containing mature human T cells is derived from a mature T cell or a cell population obtained through differentiation of pluripotent stem cells, embryonic stem cells, or cord blood stem cells.

In the preceding method, preferably, in step (1"), the human T cell is activated using an anti-human CD3 antibody, an anti-human CD28 antibody, and an anti-human CD2 antibody. In a preferred specific embodiment, the T cell is activated through incubation of magnetic beads of the anti-human CD3 antibody, the anti-human CD28 antibody, and the anti-human CD2 antibody mixed with the mature human T cell at a ratio of 1:2.

Preferably, in step (2"), the CAR molecule includes the following domains: a signal peptide, an extracellular antigen recognition domain, a transmembrane region, and an intracellular costimulatory domain. In a preferred specific embodiment, the CAR molecule includes the signal peptide, the extracellular antigen recognition domain, the transmembrane region, and the intracellular costimulatory domain in sequence from an N-terminal to a C-terminal. Preferably, the antigen is the tumor-associated antigen and/or an antigen associated with a microorganism such as a virus or a bacterium. Further preferably, the tumor-associated antigen is a tumor surface antigen, a cytokine secreted by a tumor, a surface antigen of a cell associated with immunosuppression of a tumor microenvironment and a cytokine secreted by the cell, or a tumor-associated microbial antigen, more preferably the tumor surface antigen, even more preferably CD19, GPC3, Mesothelin, PSCA, or MUC1.

Preferably, in step (2"), the BCL11B gene knockout is performed using CRISPR/CAS9 technology; further preferably, the gene knockout is performed at a second exon of a BCL11B gene; or the gene knockout is performed at a third exon of the BCL11B gene.

Preferably, in step (3"), the T cell culture medium includes IL-2; preferably, the cell obtained in step (2") is not co-cultured with OP9-DL1.

In a third aspect, the present application further provides a use of the cell according to the first aspect for preparing a drug for treatment of a disease selected from the group consisting of a tumor, AIDS, and an infectious disease; preferably, the infectious disease is a viral infectious disease.

Preferably, the drug further includes a pharmaceutically acceptable excipient.

In the present application, the human T cell is reprogrammed into an immune killer lymphocyte and the obtained immune killer lymphocyte is transfected with the CAR molecule expressing the tumor-associated antigen or the tumor-specific TCR molecule, achieving a better tumor killing effect. The reasons are as follows: the reprogrammed cell simultaneously expresses antigen recognition and killing receptors of NK cells and T cells, especially functional TCRs and have the functions of both T cells and NK cells; since the reprogrammed cell simultaneously expresses the antigen recognition and killing receptors of NK cells and T cells, the reprogrammed cell can recognize antigens sensitive to these receptors. Compared with T cells and NK cells, the reprogrammed cell not only has more extensive tumor antigen recognition and killing functions but also has more extensive functions of recognition and elimination of microorganisms such as viruses and bacteria. At the same time, the reprogrammed cell also has enhanced tumor-specific recognition and killing functions due to its CAR molecule expressing the tumor-associated antigen or its tumor-specific TCR molecule.

In addition, the engineered immune killer cell of the present application has an efficient *in vitro* proliferation ability. In adoptive cell transfer (ACT) therapy, both T cells and NK cells are used for cancer treatment. The engineered immune killer cell of the present application has the functions of both T cells and NK cells. Compared with NK cells which have limited availability and proliferation ability when applied to adoptive immunotherapy (ACT), the reprogrammed immune killer lymphocyte of the present application can be generated from a large number of T cells obtained by a user from the peripheral blood of a patient, and within 2-3 weeks, 200×10⁶ to 1248×10⁶ reprogrammed immune killer lymphocytes can be prepared and acquired from about 100×10⁶ peripheral blood mononuclear cells (PBMC) of a solid tumor patient, thereby meeting the demand of the patient for cell reinfusion.

### BRIEF DESCRIPTION OF DRAWINGS

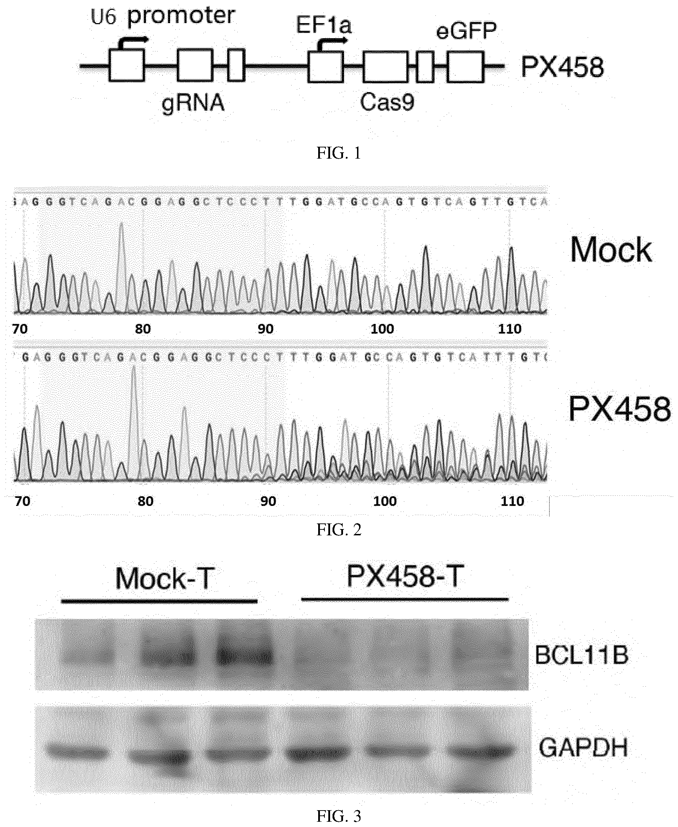

FIG. 1 is a schematic diagram of a PX458-gBCL11B vector constructed in Example 1 of the present application.
FIG. 2 is a graph showing detection and verification through gene sequencing of whether BCL11B exons of T cells transduced with PX458-gBCL11B have been knocked out, where a control group is T cells transduced with PX458 empty vectors (Mock).
FIG. 3 is a graph showing detection and verification through Western Blotting of the expression level of BCL11B proteins in T cells transduced with PX458-gBCL11B to further confirm whether the BCL11B proteins are deleted, where a control group is T cells transduced with PX458 empty vectors (Mock).

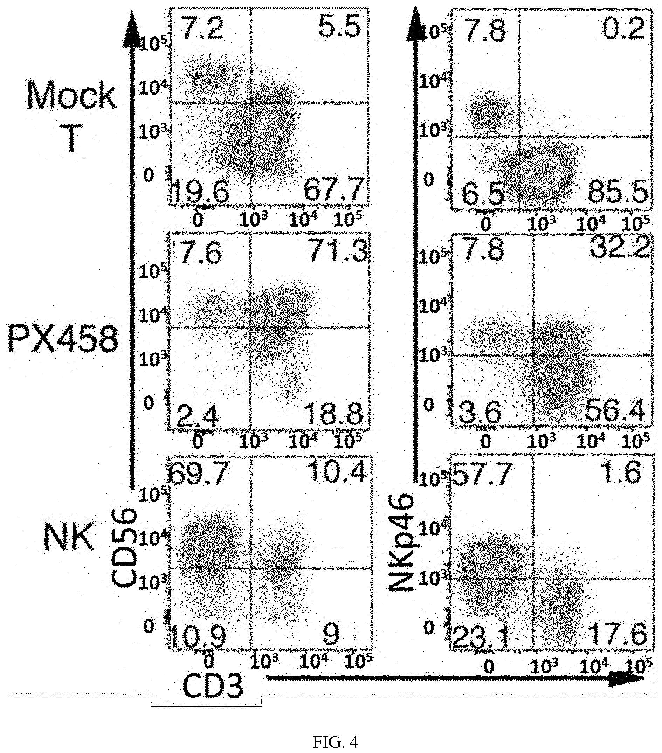

FIG. 4 is a scatter plot of flow cytometry results, which shows that compared with Mock T cells and NK cells, the ITNK cells of the present application (i.e., PX458-gBCL11B-transduced T cells, indicated by PAX458 in the figure) simultaneously perform high expression of a T cell marker CD3 and NK cell markers CD56 and NKp46. All samples are derived from the same PBMC sample, where Mock T cells are sorted from PBMCs through Pan-T, so there is 7.2% to 7.8% CD3-CD56+NKp46+NK cell mixed population; PX458 cells are obtained through the transfection of Mock T cells with PX458-gBCL11B, so there is 7.6% to 7.8% NK cell subset that is the same as that in Mock T cells; and NK cells are obtained through the culture and purification of PBMCs in an NK cell culture medium, so there is a small amount of 10.4% NKT or γδT cell mixed population of CD3+CD56+.

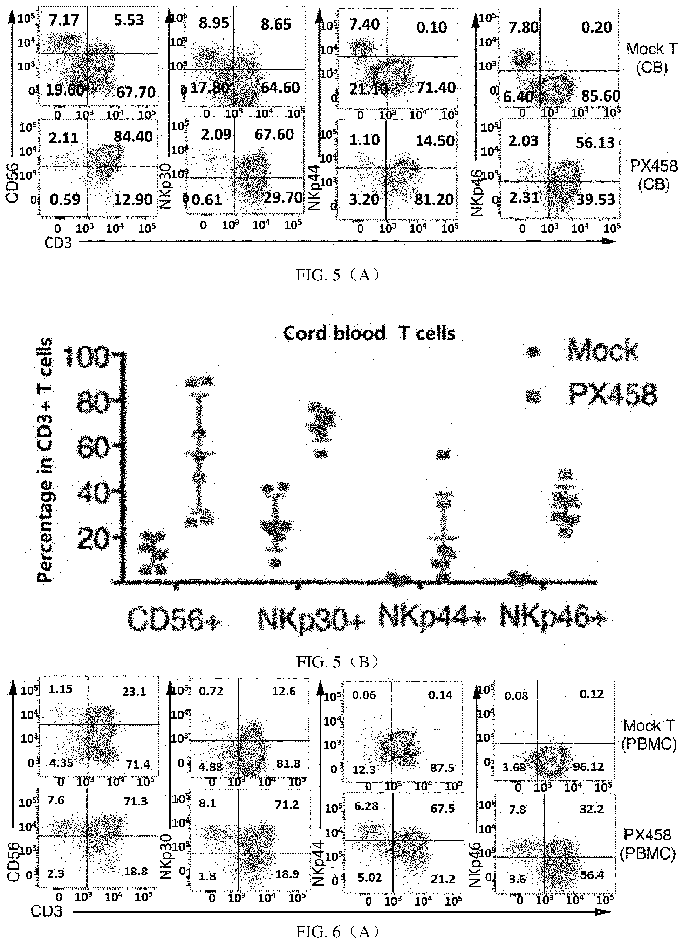

FIG. 5 is a scatter plot (A) of flow cytometry results, showing that compared with Mock T cells, T cell markers CD3 and NK cell markers NKp46, CD56, NKp30 and NKp44 are of high expression in cord blood-derived ITNK cells (i.e., PX458-gBCL11B-transduced T cells, indicated by PAX458 in the figure), and a corresponding cell percentage statistical graph (B).

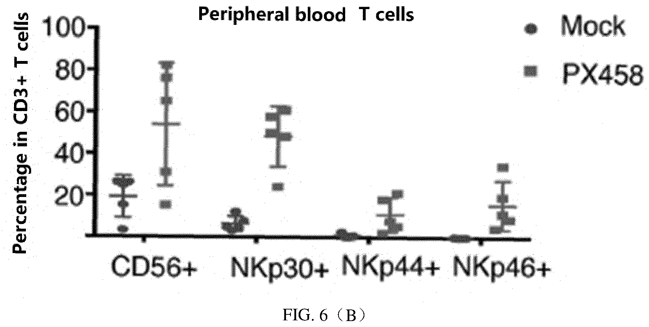

FIG. 6 is a scatter plot (A) of flow cytometry results, showing that compared with Mock T cells, T cell markers CD3 and NK cell markers NKp46, CD56, NKp30 and NKp44 are of high expression in peripheral blood-derived ITNK cells (i.e., PX458-gBCL11B-transduced T cells, indicated by PAX458 in the figure), and a corresponding cell percentage statistical graph (B).

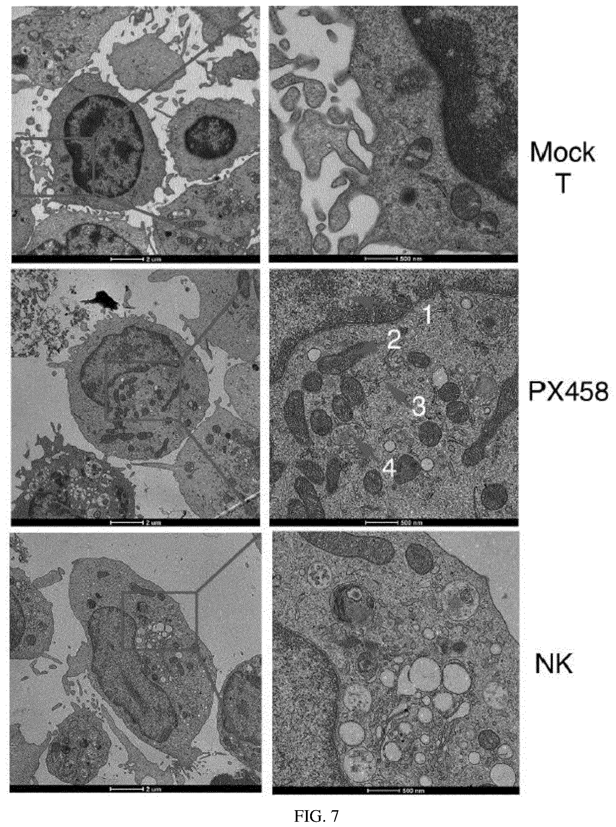

FIG. 7 is transmission electron microscopic images of Mock T cell, NK cells, and ITNK cells (i.e., PX458-gBCL11B-transduced T cells, indicated by PAX458 in the figure), which shows that an ITNK cell has lower nucleoplasm than a T cell; where 1 indicates a nucleus, 2 indicates a mitochondrion, 3 indicates endoplasmic reticulum, and 4 indicates a granule, with a scale of 2 µm in the left images and a scale of 500 nm in the right images.

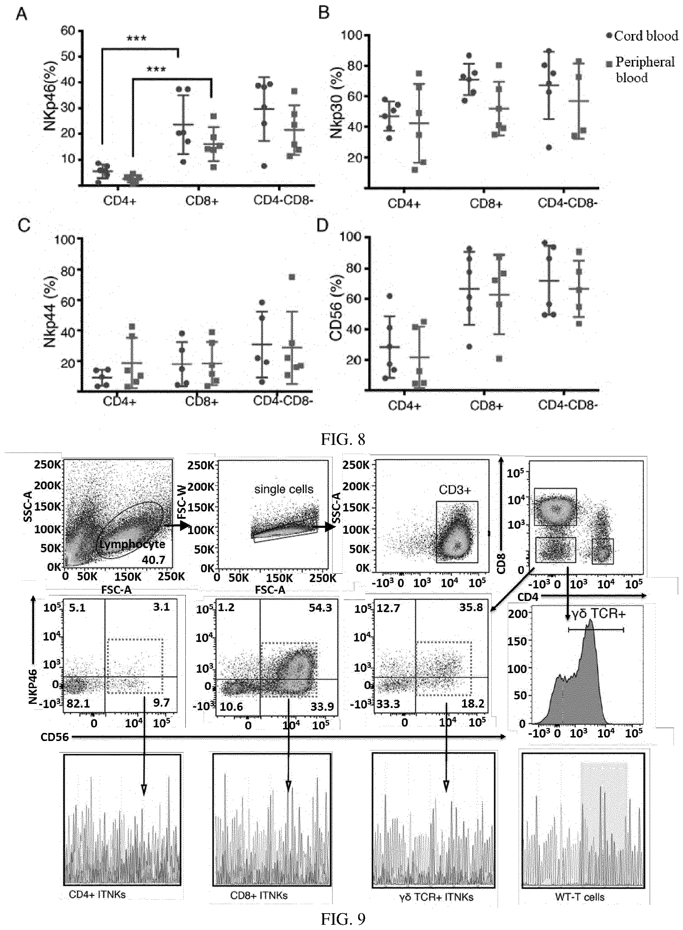

FIG. 8 shows the expression of NK receptors in CD4+, CD8+, and CD3+CD4-CD8- T cell subgroups of PX458-gBCL11B-transduced T cells (i.e., ITNK cells) derived from cord blood and peripheral blood, where the expression of NKp46 in CD8+ T cells is significantly higher than the expression of NKp46 in CD4+ T cells; n = 6, indicating that the experiment is a duplicate of 6 independent healthy donors; and ^{∗∗∗}P ≤ 0.001, and a paired t test is adopted.
FIG. 9 shows a flow cytometry analysis of PX458-gBCL11-transduced T cells, where CD56+ cells in CD4+, CD8+, and CD4-CD8- subgroups are sorted and subjected to DNA sequencing to further verify the knockout of BCL11B sites in ITNK cells.

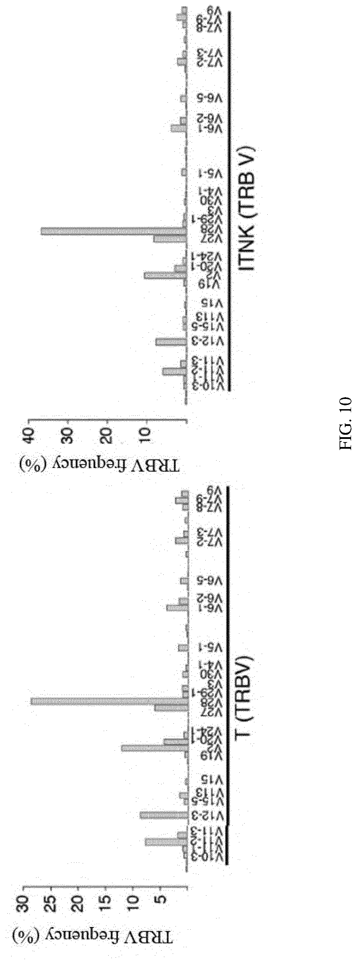

FIG. 10 shows the sequencing of TCRβ diversity, where the variable trajectory of sequences of all TCRβ chains has the same diversity in T cells and ITNK cells from the same source.

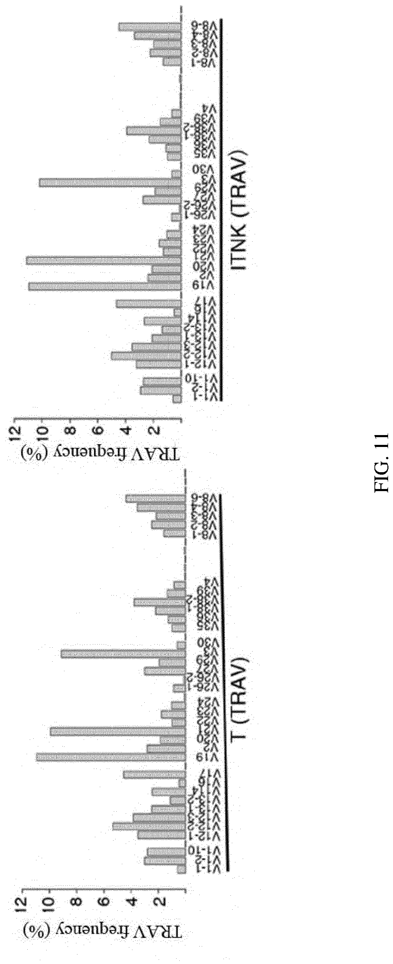

FIG. 11 shows the sequencing of TCRα diversity, where the variable trajectory of sequences of all TCRα chains has the same diversity in T cells and ITNK cells from the same source.

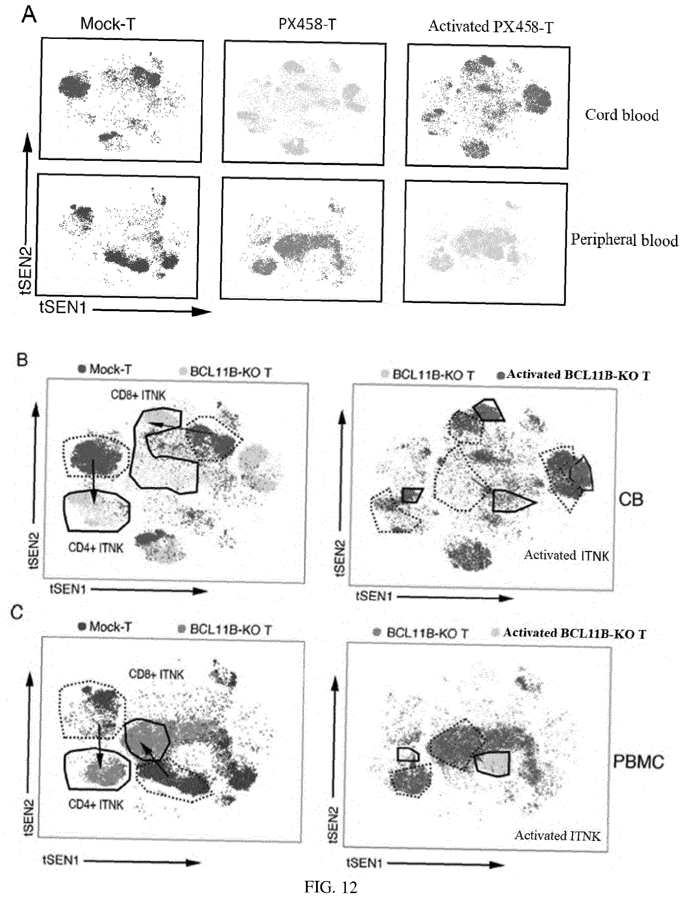

FIG. 12 is t-SEN dot density graphs of a mass cytometry clustering analysis, in which (A) shows t-SEN dot densities of CD45+ monocytes derived from cord blood and peripheral blood, respectively, where CD45+ monocytes are divided into three experimental groups: cells (Mock-T) transduced with PX458 empty vectors, cells (PX458-T) transduced with PX458-gBCL11B and cells (activated PX458-T) that are transduced with PX458-gBCL11B and then activated by K562 cells for 24 hours; and (B) and (C) show clustering differences and over-transformation (indicated by arrows respectively) between cord blood (CB)-derived Mock-T cells and CB-derived T cells with BCL11B knocked out (BCL11B-KO T), CB-derived T cells with BCL11B knocked out (BCL11B-KO T) and activated CB-derived T cells with BCL11B knocked out (Activated BCL11B-KO T), adult peripheral blood (PBMC)-derived Mock-T cells and PBMC-derived T cells with BCL11B knocked out (BCL11B-KO T), PBMC-derived T cells with BCL11B knocked out (BCL11B-KO T) and activated PBMC-derived T cells with BCL11B knocked out (Activated BCL11B-KO T), respectively.

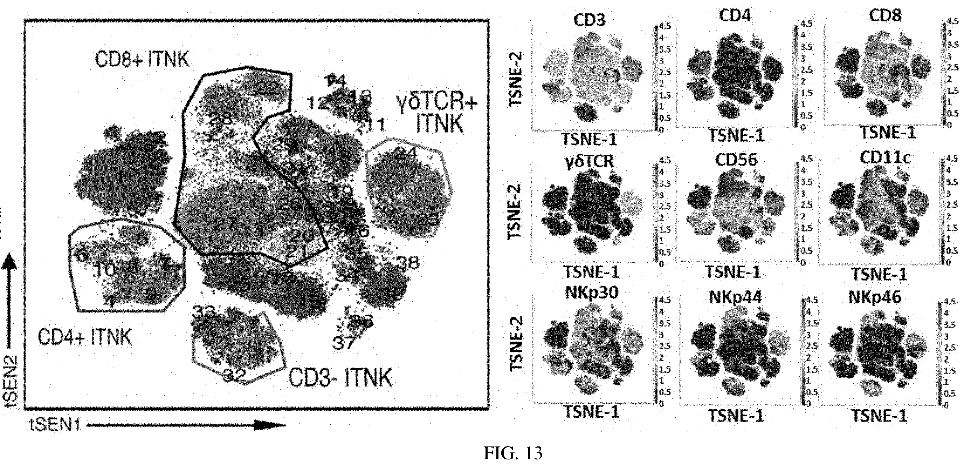

FIG. 13 is t-SEN color enrichment cluster graphs of cord blood and peripheral blood CD45+ cells fusion, where ITNK cells are marked as indicated, and cells are stained through the standardized expression of markers CD3, CD4, CD8, γδTCR CD56, NKp46, NKp30, NKp44, and CD11c on t-SEN graphs.

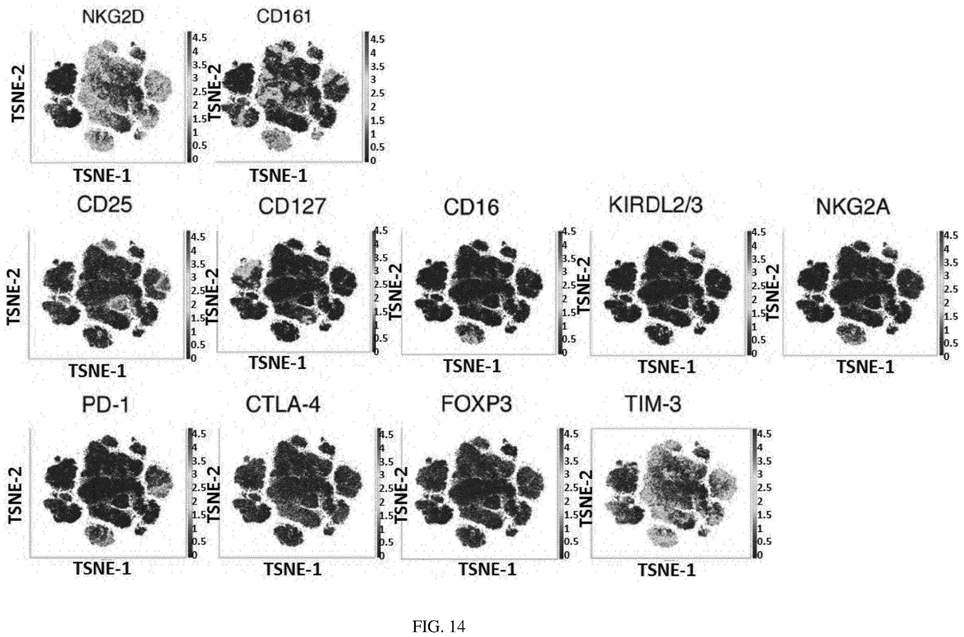

FIG. 14 is t-SEN color enrichment cluster graphs of ITNK cell immunophenotyping analysiscord blood and peripheral blood CD45+ cells fusion; where (A) shows the expression levels of markers NKG2D and CD161, (B) shows the expression levels of markers CD25, CD127, CD16, KIRDL2, KIRDL3, and NKG2A, and (C) shows the expression levels of immune checkpoint markers PD-1, CTLA-4, FOXP3, and TIM-3.

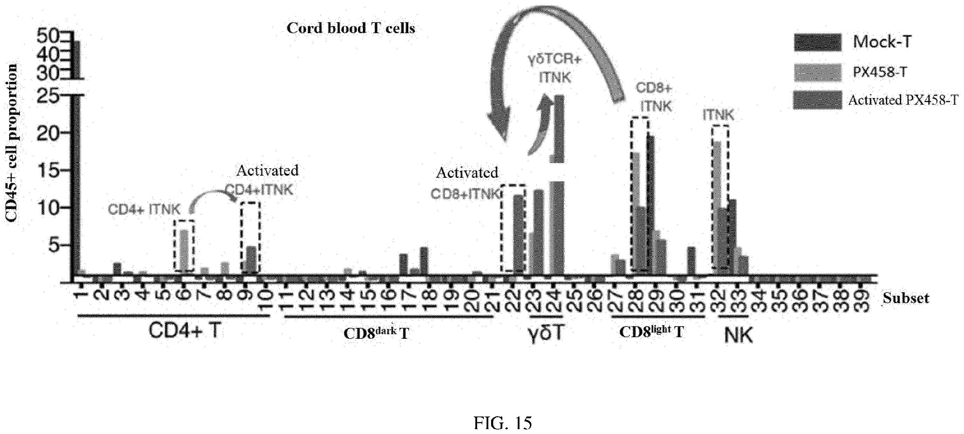

FIG. 15 is a frequency at which immune cell subgroups of T cells from cord blood are grouped, where ITNK cells can be differentiated from CD4+ T cells, CD8+ T cells, and γδTCR+ T cells, and all ITNK subtypes can be activated by HLA-negative cells (K562).

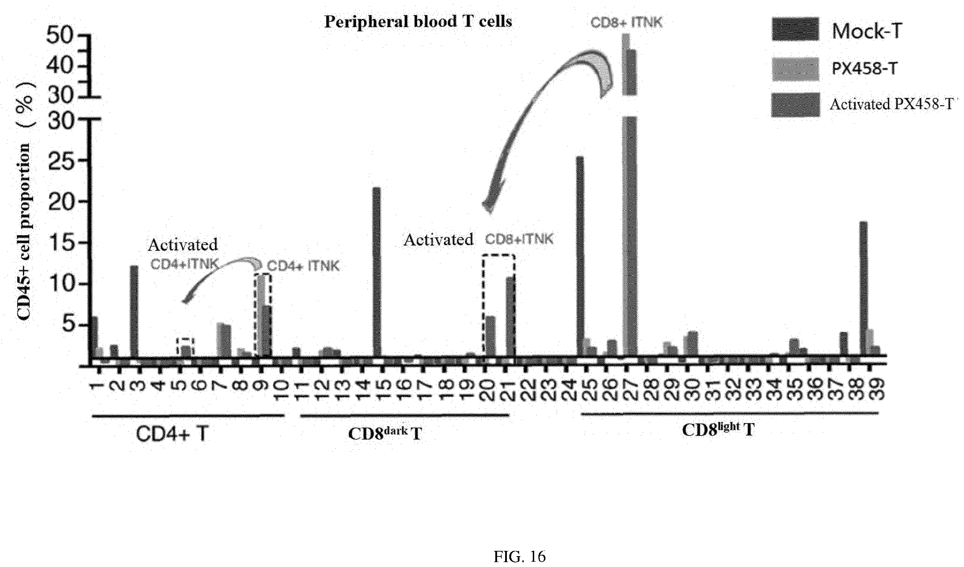

FIG. 16 is a frequency at which immune cell subgroups of T cells from peripheral blood are grouped, where ITNK cells can be differentiated from CD4+ T cells, CD8+ T cells, and γδTCR+ T cells, and all ITNK subtypes can be activated by HLA-negative cells (K562).

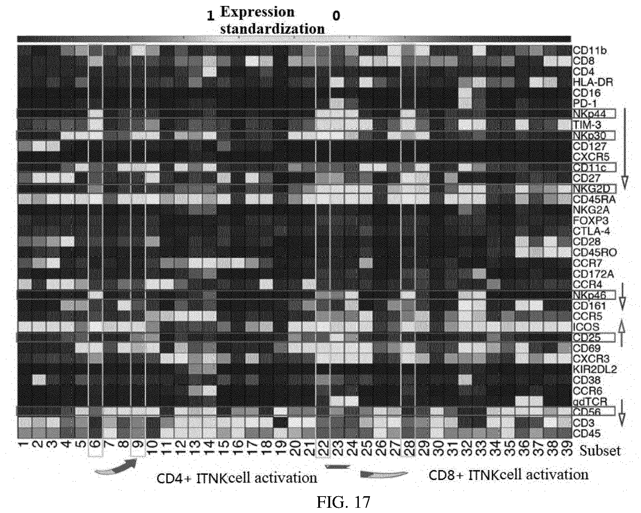

FIG. 17 shows analysis results of a mass cytometry heatmap, which shows the expression of markers of each immune cell subgroup.

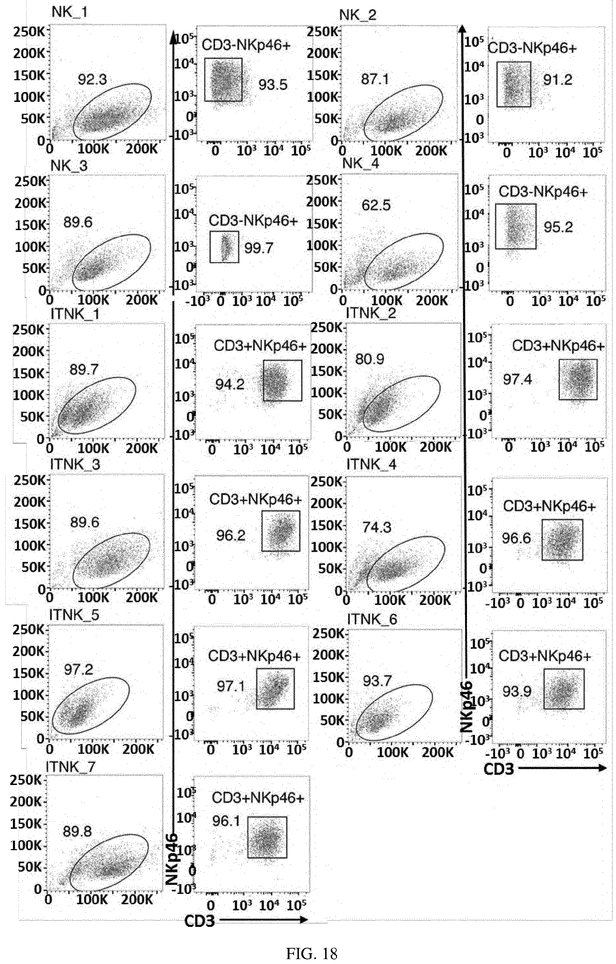

FIG. 18 shows ITNK cell sorting for RNA-Seq. CD3+ T, CD3-NKp46+ NK, CD3+NKp46+ CB-ITNK, and PBMC-ITNK in cord blood or adult peripheral blood are sorted for RNA sequencing and ATAC sequencing. The purity of sorted cells from Mock-T cells (n=6), ITNK cells (n=6), and NK cells (n=4) is 95.3±3.61%, 92.41±2.6%, and 92.88±2.06%, respectively.

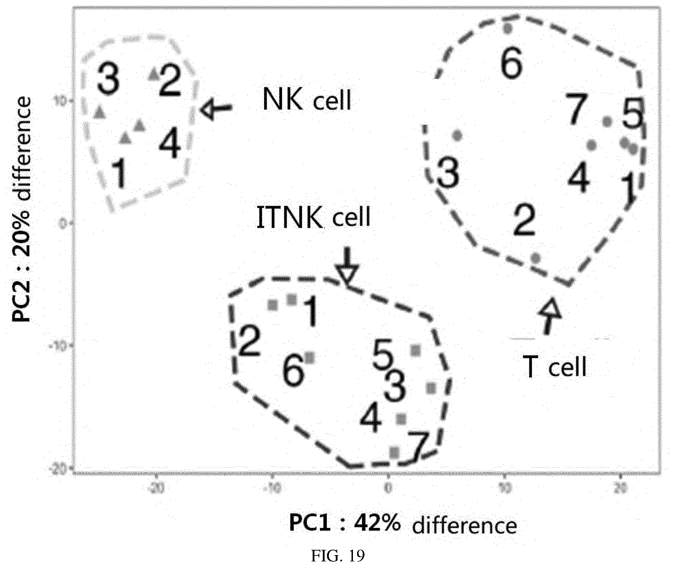

FIG. 19 is a principal component analysis of RNA sequencing-cell subgroup gene expression data.

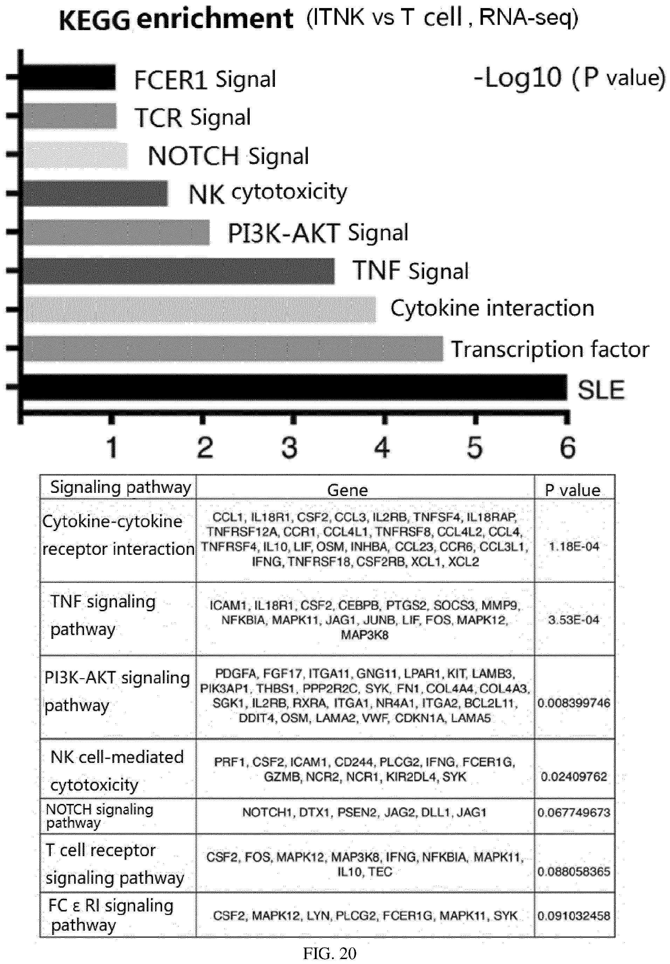

FIG. 20 is the RNA sequencing-KEGG enrichment pathway analysis of gene upregulation of ITNK cells relative to T cells (cut-off: 2 times absolute logarithm, change ≥ 1; adjusted P value ≤ 0.05).

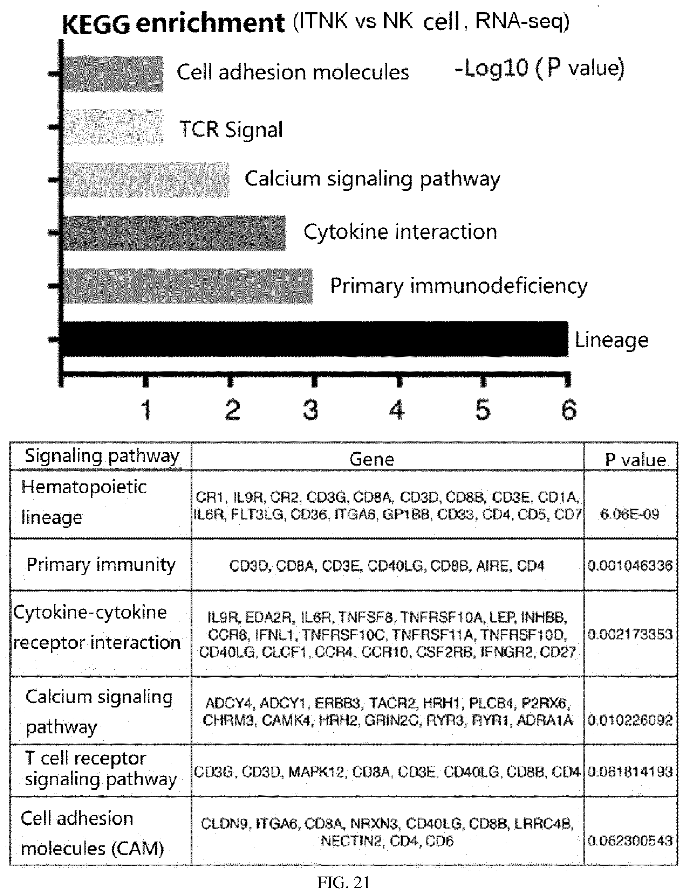

FIG. 21 is the RNA sequencing-KEGG enrichment pathway analysis of gene upregulation of ITNK cells relative to NK cells (cut-off: 2 times absolute logarithm, change ≥ 1; adjusted P value ≤ 0.05).

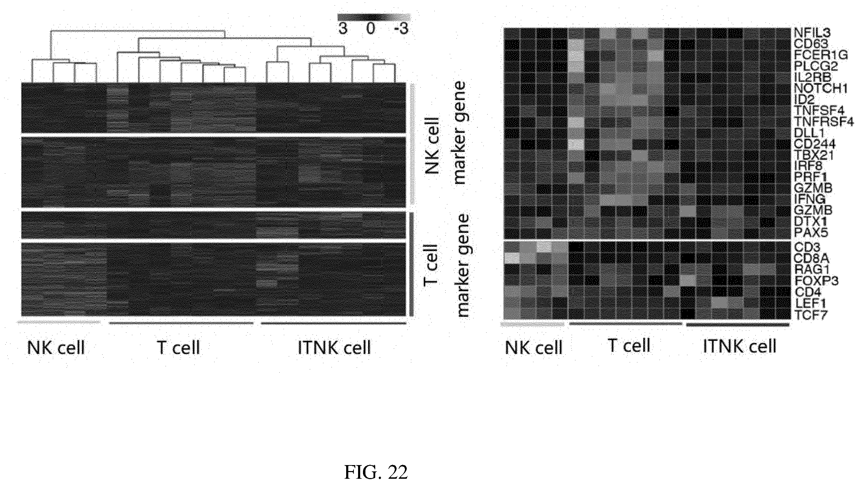

FIG. 22 is a hierarchical clustering heatmap of RNA-Seq, where the left graph shows the differences of gene transcription expression of T cells, ITNK cells, and NK cells (log2 absolute fold change ≥ 1; adjusted P value ≤ 0.05), and the right graph shows the heatmaps of differential expression of genes screened through an RNA-seq analysis in T cells, ITNK cells, and NK cells; and compared with T cells, ITNK cells upregulate the expression of NK signaling genes and downregulate the expression of TCF1 and LEF1 genes (log2 absolute fold change ≥ 1; adjusted P value ≤ 0.05).

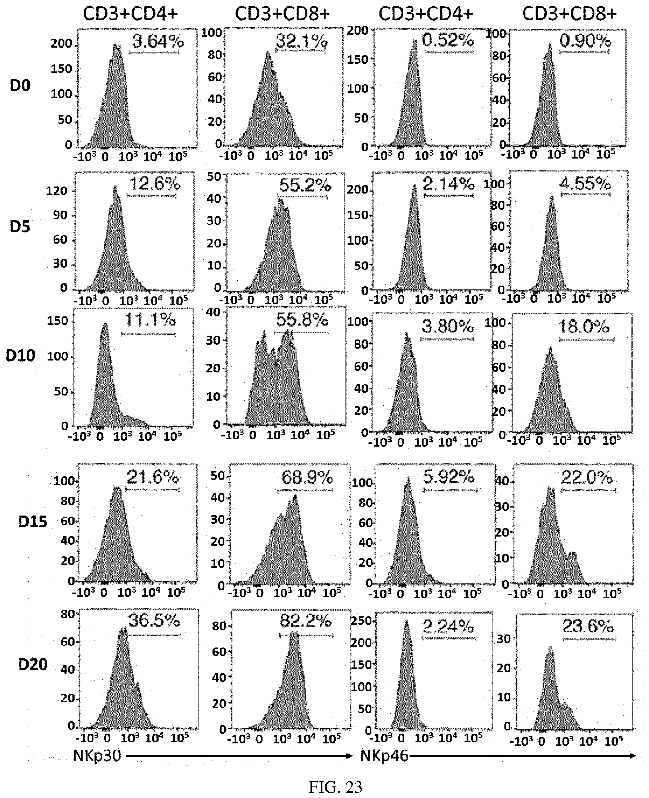

FIG. 23 is a graph of a dynamic flow cytometry analysis of immunophenotyping of ITNK cells; where the changes of the proportions of NKp30-positive and NKp46-positive subgroups in subgroups CD3+CD4+ and CD3+CD8+ are detected through flow cytometry on day 0, day 5, day 10, day 15, and day 20 after BCL11B knockout in human T cells; and the data represents three experiments; and NKp46 begins to be detected on day 5 after BCL11B knockout and stabilized on day 10 to day 15.

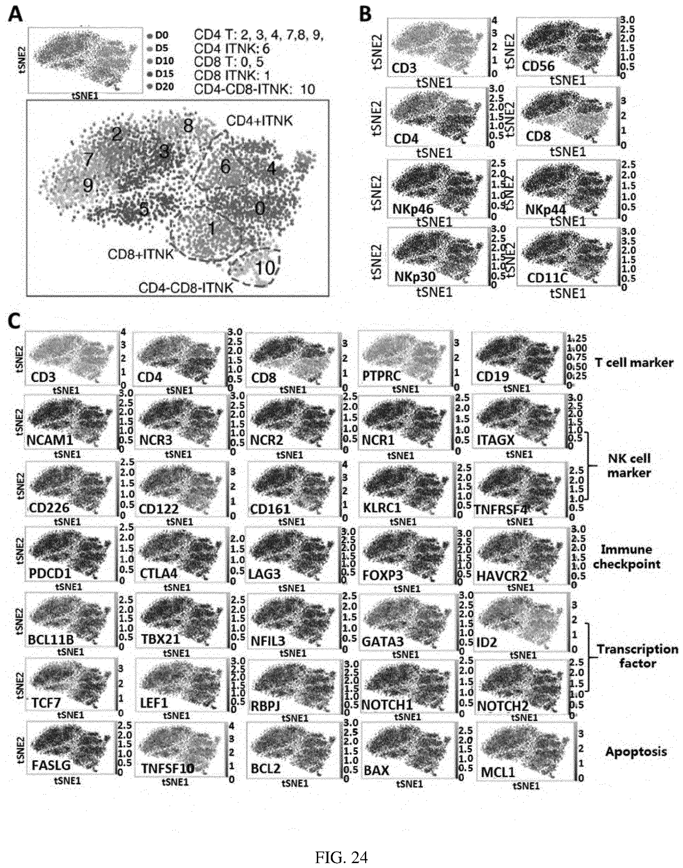

FIG. 24 (A) is a t-SNE dot plot of Sc-RNA seq analysis results, where the upper graph shows the cell distribution of D0 to D20, and the lower graph shows the clustering distribution of 4948 cells on D0(2263), D5(1565), D10(498), D15(204), and D20(418) after BCL11B knockout of T cells transduced with PX458-gBCL11B genes. The 4948 cells are clustered into 11 cell subgroups, and ITNK cells are mainly concentrated in subgroups labeled by (red) circles; (B) shows the expression of NK cell markers in the detected 4948 cells (11 subgroups) through t-SNE dot plots; (C) shows the expression of T cell markers, NK cell markers, immune checkpoints, transcription factors, and apoptosis gene-related genes in the detected 4948 cells (11 subgroups) through t-SNE dot plots.

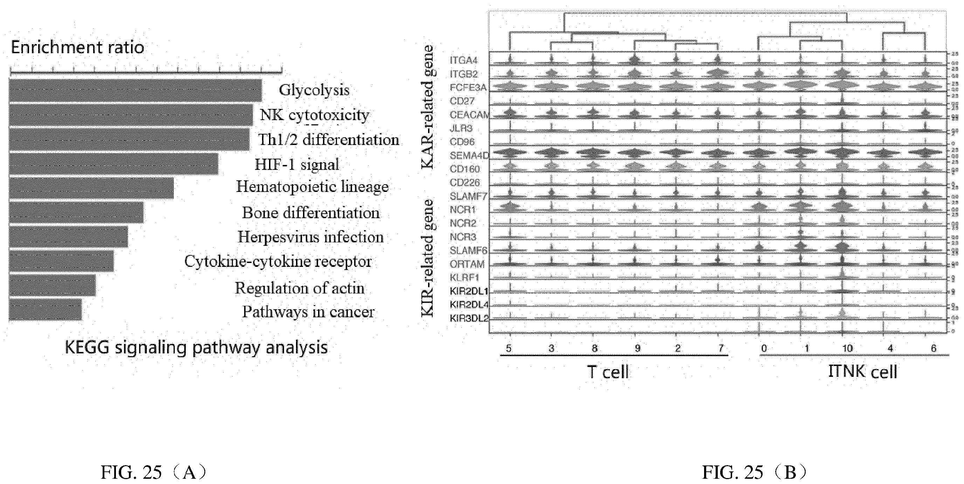

FIG. 25 (A) shows a KEGG signaling pathway analysis, which shows the high expression of NK cytotoxic genes in ITNK cells; (B) is a violin plot showing the expression profiles of KAR genes and KIR genes associated with NK cells in different subgroups; (C) is a violin plot showing the expression profiles of genes related to the development of T cells and NK cells. The expression of NK signaling genes (ID2 and TBX21), NOTCH-related genes (MXI1, ZMIZ1, and RBPJ), and AP-1-related genes (FOS, JUN, JUNB, and JUND) in ITNK cells is upregulated.

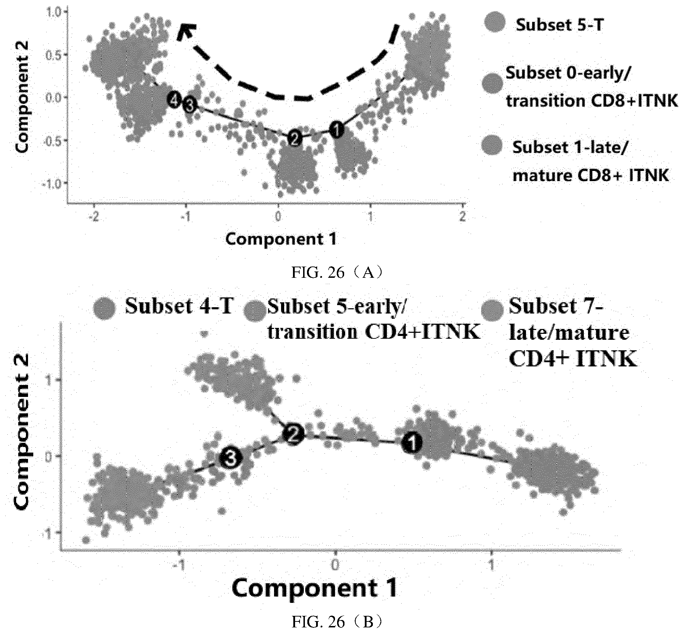

FIG. 26 (A) shows an unsupervised trajectory analysis of individual cells in subgroup 5 (CD8+ T), subgroup 0 (early CD8+ ITNK), and subgroup 1 (late CD8+ ITNK), which shows the gradual transition from CD8+ T cells to CD8+ ITNK cells; (B) shows an unsupervised trajectory analysis of individual cells in subgroup 4 (CD4+ T), subgroup 5 (early CD4+ ITNK), and subgroup 7 (late CD4+ ITNK), which shows the gradual transition from CD4+ T cells to CD4+ ITNK cells.

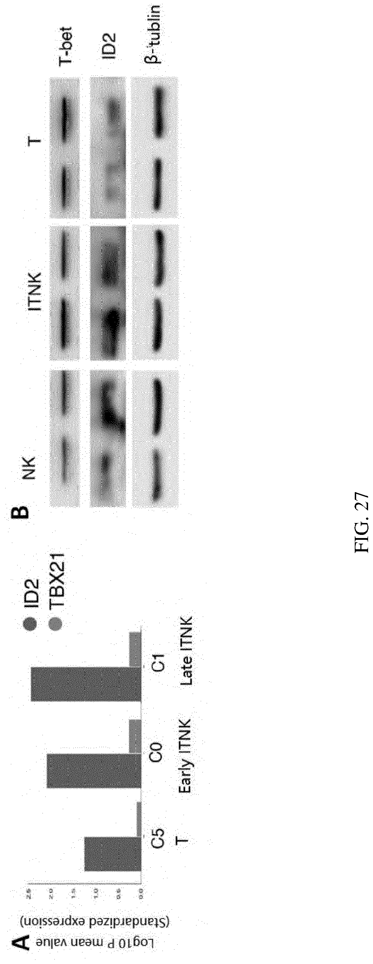

FIG. 27 (A) is a bar plot showing the upregulated expression of NK-related transcription factors ID2 and TBX21 in ITNK cells; (B) shows a Western Blotting analysis of TBX21 and ID2 in immune cell lysate, where NK cells are in the left lane, ITNK cells are in the middle lane, and T cells are in the right lane, with β-Tublin as internal reference.

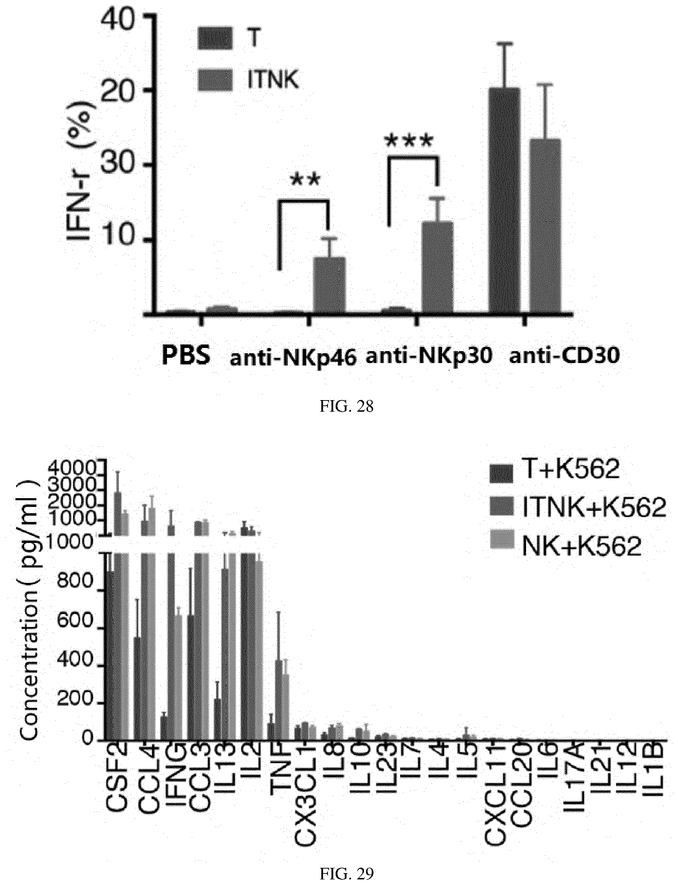

FIG. 28 is a histogram showing the detection through an ELISA of IFNy secreted by T cells and ITNK cells which are stimulated with an anti-NKp30 antibody, an anti-NKp46 antibody, and an anti-CD3/CD28 antibody (5 ug/mL), separately, where the data is from samples of three independent donors; the data is represented as mean±SD; ^{∗∗}P ≤ 0.01, and ^{∗∗∗}P ≤ 0.001; and a paired t test is adopted.
FIG. 29 shows the secretion of cytokines in T cells, ITNK cells and NK cells which are stimulated by K562 cells. Specifically, T cells, ITNK cells, and NK cells are incubated with K562 cells at 37 °C for 18 hours at an effector (E):target (T) ratio of 1:1, separately; the supernatant is collected and the concentrations of cytokines (CSF2, CCL4, IF γ, CCL3, IL13, IL2, TNF, CX3CL1, IL8, IL10, IL23, IL7, IL4, IL5, CXCL11, CCL20, IL6, IL17A, IL21, IL12, and IL1 β) are measured through a multiplex immunoassay; where the value is expressed as the mean of three different donors±SD.

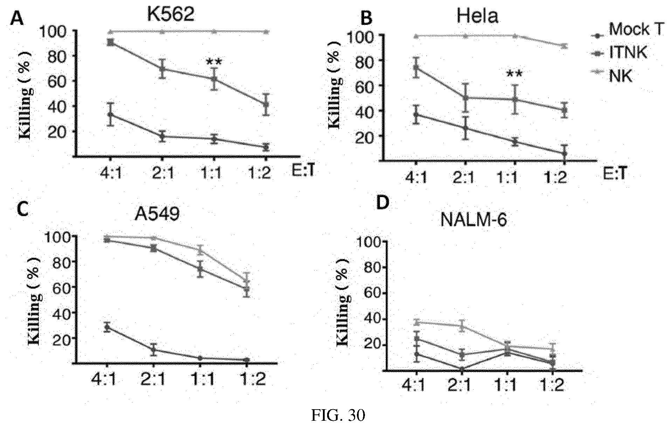

FIG. 30 shows the specific cytotoxicity percentages of ITNK cells to HLA-negative K562 cells (A), HLA-positive Hela cells (B), HLA-positive A549 cells (C), and HLA-positive NALM-6 cells (D), where the data is expressed as mean±SD; ^{∗∗}P 0.01 0.01; an unpaired t-test is adopted.

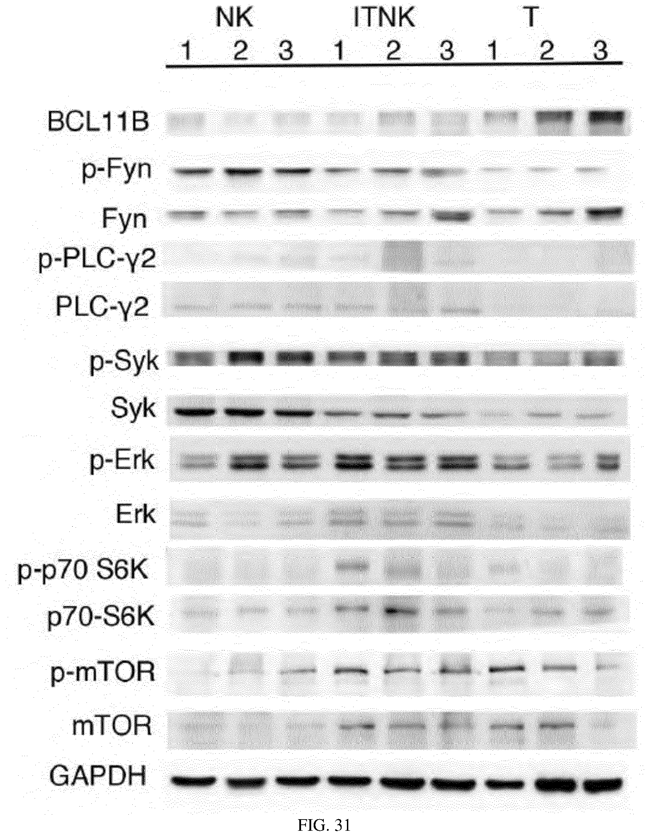

FIG. 31 shows the protein and phosphorylation levels, which are measured through immunoblotting, of Fyn, PLC-g2, Syk, Erk1/2, and mTOR in the lysate of immune cells stimulated by K562 cells for 6 hours, wherein NK cells are in the left three lanes, ITNK cells are in the middle three lanes, and T cells are in the right three lanes, with BCL11B as gene editing control and GAPDH as sample control.

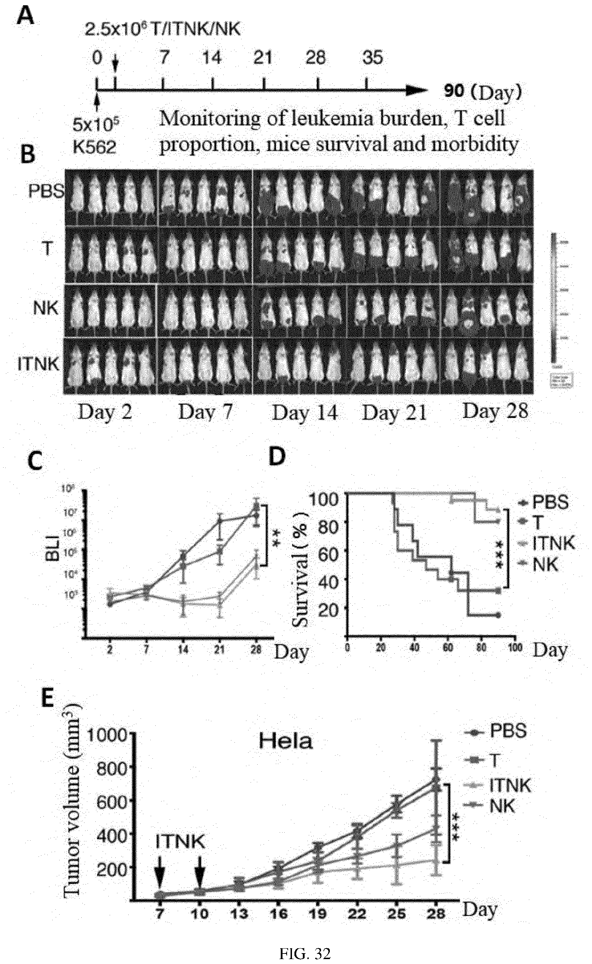

FIG. 32 (A) is a schematic diagram of an assay for detecting ITNK cells that kill tumor cells *in vivo;* (B) and (C) show a quantitative analysis of the total flux of luciferase activity in experimental mice at particular time points through *in vivo* bioluminescence imaging (5 mice in each group), where the result is mean±SD, ^{∗∗}P≤0.01, and an unpaired t test is adopted; (D) is a statistical graph of survival time of K562 tumor-bearing mice treated with PBS (n=10), Mock T (n=15), ITNK (n=15), and NK cells (n=5); (E) shows that Hela tumor-bearing mice are treated on day 7 and day 10 with PBS, Mock T cells, ITNK cells, and NK cells respectively and the size of tumor is detected at designated time points (5 mice in each group), where the data is expressed as mean±SD, ^{∗∗∗}P≤0.001, and an unpaired t test is adopted.

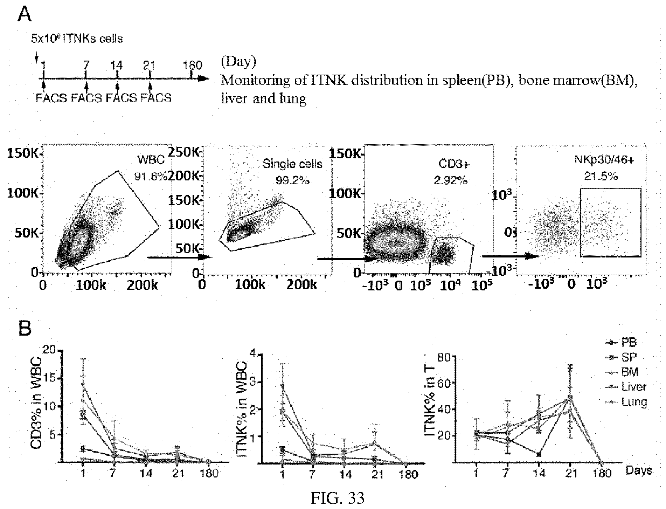

FIG. 33 shows the distribution and maintenance of ITNK cells in mice after the ITNK cells are transplanted into NSI-strain immunodeficient mice lacking T cells, B cells, and NK cells, wherein, the top graph in (A) is a schematic diagram of the injection of ITNK cells into NSI-strain mice and the detection of ITNK cells, which shows that ITNK cells are subjected to a short-term analysis and a long-term analysis on day 1, day 7, day 14, day 21, and in a sixth month, respectively (n=3 for each group); the lower graph in (A) shows the percentages of ITNK cells in CD3+ T cells, which are analyzed through representative flow cytometry; and (B) shows the dynamic distribution of ITNK cells in peripheral blood (PB), bone marrow (BM), spleen, liver, and lung of mice, which shows that no T cells and no ITNK cells can be detected 6 months after the injection of ITNK cells.

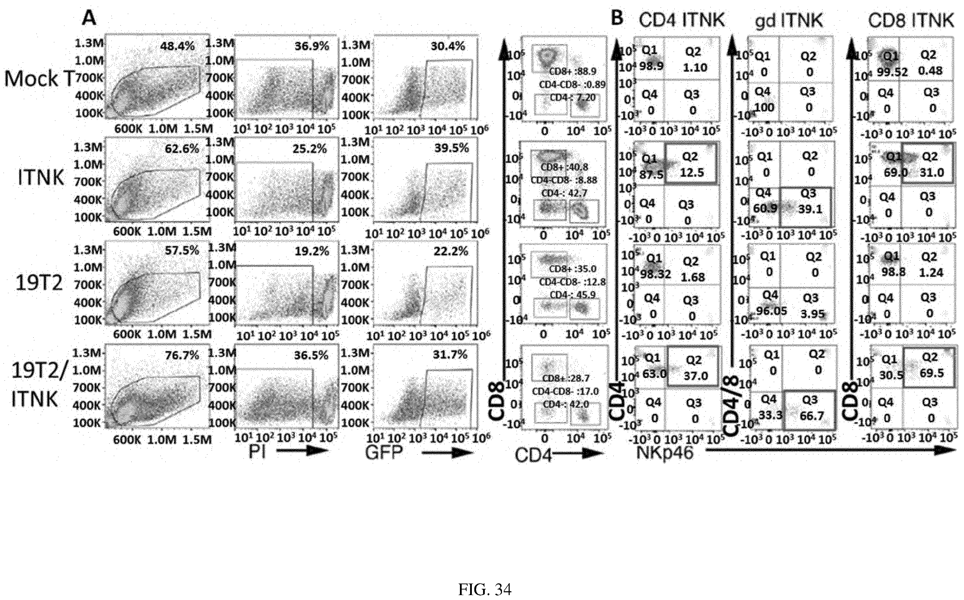

FIG. 34 shows the PI labels and GFP expression of the transduced cells, which is detected through flow cytometry, to determine the survival and transduction efficiency of the cells after transduction (as shown in Figure 34 A), and the reprogramming of ITNK cells (B).

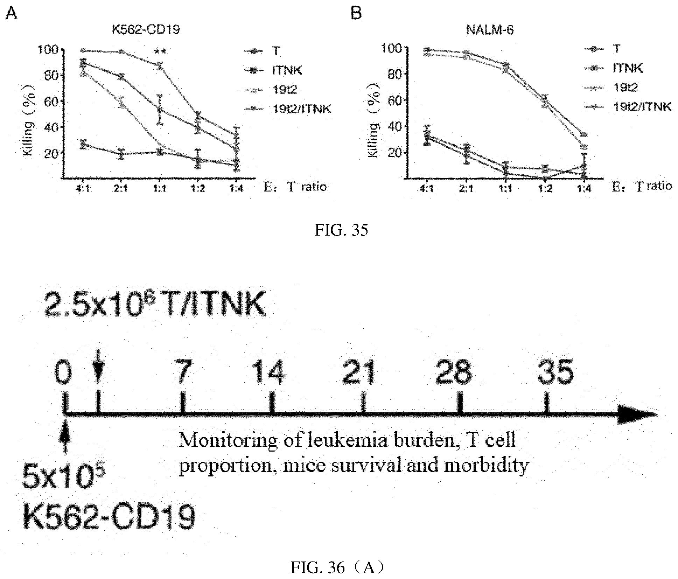

FIG. 35 shows the lysis rates of targeted tumor cells when each group of effector cells (T, ITNK, 19t2, and 19t2/ITNK) is co-cultured with target cells K562-CD19 cells (A) and NALM6 cells (B) at different number ratios of effector cells to target cells (E:T ratios), which are expressed as mean±SD; ^{∗∗}P≤0.01; and an unpaired t test is adopted.

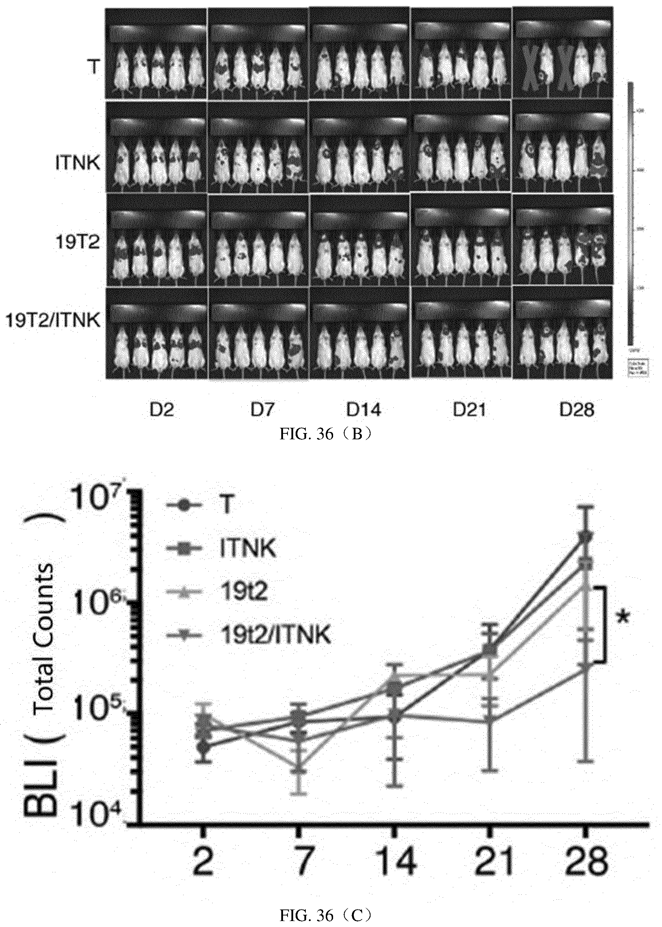

FIG. 36A is a schematic diagram showing the experiment in which NSI mice are intravenously injected with CD19+K562-GL cells (5×10⁵), where the mice are injected with T cells, ITNK cells, and NK cells (2.5×10⁶) on day 2, and bioluminescence imaging is performed on day 2, day 7, day 14, day 21, and day 28, separately (5 mice in each group); FIG. 36 (B) is a statistical plot of a quantitative analysis of the total flux of luciferase activity through *in vivo* bioluminescence imaging; and FIG. 36 (C) shows an image of living bodies (5 mice in each group). The results are expressed as mean±SD; ^{∗}P ≤ 0.05, an unpaired t test is adopted for designated time points.

### DETAILED DESCRIPTION

To further elaborate on the technical means adopted and the effects achieved in the present application, the technical solutions of the present application are further described below through specific examples in conjunction with drawings. However, the present application is not limited to the scope of the examples.

Unless otherwise stated, the present application is not limited to the relative arrangement, numeric expressions and numerical values of the components and steps set forth in these examples. The techniques, methods, and devices known to those of ordinary skill in the art may not be discussed in detail, but in appropriate circumstances, the techniques, methods, and devices should be regarded as part of the specification.

### Example 1 Preparation of reprogrammed natural killer (ITNK) cells of the present application

### Construction of a gene knockout plasmid vector

According to the selection rule of a CRISP/CAS9 target site: GN19NGG, where GN19 was a target site, N was better G, and the target site can be on an antisense strand (that is, the sequence on a sense strand is CCN N19C), the following target sequences were selected and forward (F) and reverse (R) primers were designed separately as guideRNA (gRNA). The gRNA was annealed and ligated into the digested PX458 vector to construct a PX458-gBCL11B vector (as shown in FIG. 1). PX458-gBCL11B was transfected in the 293 T cell line, and monoclones were selected. The knockout conditions such as base deletion and dislocation of BCL11B knockout targets were detected through gene sequencing (see FIG. 2), and the knockout efficiency was statistically calculated, as shown in Table 1 below.

**Table 1 List of BCL11B target sequences and their corresponding gRNA**

| Location | Target Sequence | Forward and Reverse Primers | Speci ficity | Knoc kout Effici ency |
|---|---|---|---|---|
| Second exon | GACCCTGACCTG CTCACCTG (SEQ ID NO: 1) | F: | 58 | 39 |
| | | caccGACCCTGACCTGCTCACCTG (SEQ ID NO: 2) | | |
| | | R: | | |
| | | aaacCAGGTGAGCAGGTCAGGGTC (SEQ ID NO: 3) | | |
| Second exon | GAAGCAGTGTG GCGGCAGCT (SEQ ID NO: 4) | F: | 67 | 48 |
| | | caccGAAGCAGTGTGGCGGCAGCT (SEQ ID NO: 5) | | |
| | | R: | | |
| | | aaacAGCTGCCGCCACACTGCTTC (SEQ ID NO: 6) | | |
| Second exon | CAGGTGGTCATC TTCGTCGG (SEQ ID NO: 7) | F: | 97 | 90 |
| | | caccCAGGTGGTCATCTTCGTCGG (SEQ ID NO: 8) | | |
| | | R: | | |
| | | aaacCCGACGAAGATGACCACCTG (SEQ ID NO: 9) | | |
| Second exon | GCAGGTGGTCAT CTTCGTC (SEQ ID NO: 10) | F: caccGCAGGTGGTCATCTTCGTC (SEQ ID NO: 11) | 95 | 60 |
| | | R: | | |
| | | aaacCGACGAAGATGACCACCTG (SEQ ID NO: 12) | | |
| Second exon | GCTCAGGAAAGT GTCCGAGC (SEQ ID NO: 13) | F: | 86 | 59 |
| | | caccGCTCAGGAAAGTGTCCGAGC (SEQ ID NO: 14) | | |
| | | R: | | |
| | | aaacGCTCGGACACTTTCCTGAG (SEQ ID NO: 15) | | |
| Third exon | GAGTCCCGTCAC CCGAGACC (SEQ ID NO: 16) | F: | 93 | 49 |
| | | caccGAGTCCCGTCACCCGAGACC (SEQ ID NO: 17) | | |
| | | R: | | |
| | | aaacGGTCTCGGGTGACGGGACT (SEQ ID NO: 18) | | |
| Third exon | GAAGTGA TCACG GATGAGTG (SEQ ID NO: 19) | F: | 81 | 55 |
| | | caccGAAGTGATCACGGATGAGTG (SEQ ID NO: 20) | | |
| | | R: aaacCACTCATCCGTGATCACTT (SEQ ID NO: 21) | | |
| Third exon | GGTGACGGGACT CAGGGTGA (SEQ ID NO: 22) | F: | 62 | 69 |
| | | caccGGTGACGGGACTCAGGGTGA (SEQ ID NO: 23) | | |
| | | R: | | |
| | | aaacTCACCCTGAGTCCCGTCAC (SEQ ID NO: 24) | | |
| Third exon | TGCAGCGCGCGC CCGGTCTC (SEQ ID NO: 25) | F: | 87 | 63 |
| | | caccTGCAGCGCGCGCCCGGTCTC (SEQ ID NO: 26) | | |
| | | R: | | |
| | | aaacGAGACCGGGCGCGCGCTGCA (SEQ ID NO: 27) | | |
| Fourth exon | CACGAGAGCGA CCCGTCGCT (SEQ ID NO: 28) | F: | 99 | 49 |
| | | caccCACGAGAGCGACCCGTCGCT (SEQ ID NO: 29) | | |
| | | R: | | |
| | | aaacAGCGACGGGTCGCTCTCGTG (SEQ ID NO: 30) | | |
| Fourth exon | GCGACGGGTCGC TCTCGTGG (SEQ ID NO: 31) | F: | 97 | 69 |
| | | caccGCGACGGGTCGCTCTCGTGG (SEQ ID NO: 32) | | |
| | | R: | | |
| | | aaacCCACGAGAGCGACCCGTCG (SEQ ID NO: 33) | | |
| Fourth exon | TCCATGCTGAAG CTCGACTC (SEQ ID NO: 34) | F: | 91 | 60 |
| | | caccTCCATGCTGAAGCTCGACTC (SEQ ID NO: 35) | | |
| | | R: | | |
| | | aaacGAGTCGAGCTTCAGCATGGA (SEQ ID NO: 36) | | |
| Fourth exon | ACGGGTCGCTCT CGTGGTGG (SEQ ID NO: 37) | F: | | 92 |
| | | caccACGGGTCGCTCTCGTGGTGG (SEQ ID NO: 38) | | |
| | | R: | 90 | |
| | | aaacCCACCACGAGAGCGACCCGT (SEQ ID NO: 39) | | |
| Fourth exon | AGCCGCAACCGC GAGAACGG (SEQ ID NO: 40) | F: | 98 | 55 |
| | | caccAGCCGCAACCGCGAGAACG G (SEQ ID NO: 41) | | |
| | | R: | | |
| | | aaacCCGTTCTCGCGGTTGCGGCT (SEQ ID NO: 42) | | |
| Fourth exon | GCAACTTGACGG TGCACCGG (SEQ ID NO: 43) | F: | 97 | 73 |
| | | caccGCAACTTGACGGTGCACCGG (SEQ ID NO: 44) | | |
| | | R: | | |
| | | aaacCCGGTGCACCGTCAAGTTG (SEQ ID NO: 45) | | |
| Fourth exon | GAGCTGGGCCGC CCGGGGCC (SEQ ID NO: 46) | F: | 39 | 76 |
| | | caccGAGCTGGGCCGCCCGGGGCC (SEQ ID NO: 47) | | |
| | | R: | | |
| | | aaacGGCCCCGGGCGGCCCAGCT (SEQ ID NO: 48) | | |
| Third exon | GGTCAGACGGA GGCTCCCTT (SEQ ID NO: 49) | F: | 61 | 65 |
| | | caccGGTCAGACGGAGGCTCCCTT (SEQ ID NO: 50) | | |
| | | R: | | |
| | | aaacAAGGGAGCCTCCGTCTGACC (SEQ ID NO: 51) | | |

According to the knockout efficiency in Table 1, the gRNA gene knockout plasmid vectors with knockout at the second exon and the third exon were selected for the next step. In the present application, BCL11B gene knockout was preferably performed at the second exon and the third exon, and gene knockout plasmids corresponding to a mixture of a first pair of gRNA and a second pair of gRNA with the lowest knockout efficiency at the second exon, a third pair of gRNA with the highest knockout efficiency at the second exon, and a third pair of gRNA with the lowest knockout efficiency at the third exon and a mixture thereof can all reprogram T cells into immune killer lymphocytes of the present application. In this example, BCL11B gene knockout plasmids were constructed by using gRNA of SEQ ID NO: 5, SEQ ID NO: 6, SEQ ID NO: 50 and SEQ ID NO: 51, respectively and mixed for the next step.

### T cell sorting and activation

T cells were sorted and activated by the following method:
(1) peripheral blood and cord blood including mature human T cells were centrifuged at 300×g for 10 minutes, separately, and plasma was collected and thermally inactivated at 56°C for 30 minutes;
(2) the precipitated granular blood cells were suspended with 0.9% NaCl, and peripheral blood mononuclear cells (PBMCs) were separated through Ficoll density gradient centrifugation; and
(3) negative sorting was performed with an MACS Pan T separation kit (produced by Miltenyi Biotec in Bergisch Gladbach, Germany) to enrich all T cells (Pan T) from the blood such as peripheral blood and cord blood.

The above (1) to (3) were the steps of isolating mature human T cells from peripheral blood and cord blood. It is to be noted that other T cell sources are also acceptable, such as the committed differentiation of pluripotent stem cells and hematopoietic stem cells. T cells from all sources were activated with a T cell activation kit (produced by Miltenyi Biotec) through the incubation of magnetic beads coated with anti-human CD3, anti-human CD28, and anti-human CD2 antibodies mixed with T cells at a ratio of 1:2 (cell density: 2.5×10⁶ cells/mL, and culture medium: T551-H3 (produced by Takara, Japan) containing 5% autologous plasma, hIL2 (100 IU/mL), gentamicin sulfate (20 µg/mL), 10 mm of HEPES, 2 mm of glutamine, and 1% penicillin/streptomycin). Having been activated for 24-48 hours, T cells were eluted from antibiotin MACS iBead TM granules for later use.

### Induced reprogramming

(1) CRISP/CAS9 knockout vectors PX458-gBCL11B were transduced into the above-mentioned activated T cells by an electrotransfer (T-023, LONZA Amaxa Nucleofector, Lonza);
(2) after 12 hours, T cells transduced with PX458-gBCL11B (such cells were simply referred to as PX458-T) were centrifuged and cultured in a T551-H3 (produced by Takara, Japan) medium (containing 5% autologous plasma or fetal bovine serum (FBS), 500 IU/mL hIL2 and gentamicin sulfate (20 µg/mL));
(3) a fresh medium was changed every three days and the cell density was kept within a range of 0.5×10⁶ cells/mL to 1×10⁶ cells/mL until electroporation was performed for 14 days;
(4) whether the second exon or the third exon of BCL11B of the T cells transduced with PX458-gBCL11B was subjected to knockout, such as induced insertion or deletion of sites, was detected and verified through gene sequencing; where the control group was T cells transduced with PX458 empty vectors (Mock); and
(5) the expression level of BCL11B proteins in the T cells transduced with PX458-gBCL11B was detected and verified through Western Blotting to further confirm the deletion of BCL11B proteins, where the control group was the T cells transduced with PX458 empty vectors (Mock). The results of Western Blotting are shown in FIG. 3.

### Phenotype identification of reprogrammed cells

As described above, after T cells were subjected to electroporation for 14 days, 19.5% to 68.7% of the resulting cells expressed both T cell markers such as CD3 and NK cell markers such as NKp46, CD56, NKp30, and NKp44, and then it was determined that the human ITNK cells of the present application were obtained. NK cells expressed only NK cell markers such as NKp46 and CD56 and did not express T cell markers such as CD3. The T cells electroporated with empty vectors expressed T cell markers such as CD3 and did not express NK cell markers. The expression of cell markers of T cells, NK cells, and ITNK cells is shown in FIGS. 4 to 6, and their phenotypic differences are summarized in Table 2 below.

**Table 2 Phenotypic differences of ITNK cells, T cells, and NK cells**

| -- | T Cell | NK Cell | ITNK Cell |
|---|---|---|---|
| T cell marker | CD3, etc. | - | CD3, etc. |
| NK cell marker | - | High expression of NKp46, CD56, NKp30, NKp44, and the like | High expression of NKp46 (CD4-positive ITNK cells perform low/no expression), CD56, NKp30, NKp44 and the like |
| BCL11B expression | High expression | Low expression | Deletion |

In addition, it shows through the observation with a confocal microscope that the cell morphology of an ITNK cell reprogrammed from the T cell is different from that of the T cell and similar to that of an NK cell, and the reprogrammed ITNK cell has a relatively small nucleus (relative to a volume of the nucleus of the T cell which occupies the whole cell), a relatively plentiful intracellular matrix, a larger granule, abundant endoplasmic reticulum, and high protein synthesis activity, indicating that the reprogrammed ITNK cell is an immune killer lymphocyte. The transmission electron microscopic images of the T cell, the NK cell, and the ITNK cell are shown in FIG. 7.

In addition, the inventors also compared the expression profiles of these NK markers in BCL11B-deficient T cell subgroups derived from cord blood and peripheral blood and found that the percentages of CD8+NKp46+ cells and CD8+CD56+ cells were significantly higher than the percentages of CD4+NKp46+ cells and CD4+CD56+ cells, indicating that NKp46+CD3+ ITNK cells were mainly derived from CD8+ T cells (see FIG. 8). Unlike CD8+ T cells, CD4+ T cells expressed NKp30 and did not express NKp46 after the deletion of BCL11B (see FIG. 8B).

The CD4-CD8-NKp46+ subgroup expressed "TCRyδ" and was γδTCR+ITNK cells (see FIG. 9). The deletion of BCL11B in CD4+ T cells, CD8+ T cells, and γδTCR+T cells was further verified through DNA sequencing (see FIG. 9).

### Example 2 Source identification of ITNK cells of the present application

TCRαβ sequencing: T cells and ITNK cells obtained in Example 1, both of which were derived from the same donor, were subjected to RNA extraction and CDR3 region targeted proliferation through a human TCR-αβ analysis kit to obtain TCR RNA. TCR RNA was sequenced on Hiseq 4000 platform to obtain a TCR library. A clustering combination analysis was performed with MiXCR(ref). The types of TCRαβ clones were derived with the parameter of "-chain" through MiXCR clone derivation instructions. The diversity of TCR clones of T cells and ITNK cells which were both derived from the same donor was compared through TCR sequencing. It was found that the diversity of TCR clones was consistent (see FIGS. 10 and 11). Therefore, it was determined that the resulting ITNK cells were reprogrammed from T cells with BCL11B deleted and maintained the TCR diversity of the T cells instead of being proliferated from a special and unknown small subgroup of human T cells.

### Example 3 Single-cell immunophenotyping identification of ITNK cells of the present application

The ITNK cells obtained in Example 1 were subjected to a single-cell immunophenotyping analysis through mass cytometry (CyTOF), separately. The control group was T cells transduced with empty vectors.

Preparation and pretreatment of mass spectrometer samples: Cells from a culture suspension were centrifuged, re-suspended with PBS containing 0.5% BSA and 0.02% NaN3, and incubated with an anti-human CD16/32 monoclonal antibody at room temperature for 10 minutes to block an Fc receptor. Then, a mixture of metal-labeled antibodies against cell surface molecules was added and further incubated on ice for 20 minutes. The antibodies were pre-coupled antibodies (produced by Fluidigm) or were internally coupled using a mass spectrometry flow coupling kit (produced by Fluidigm) according to the instructions. 5 mM of cisplatin was added to the cells, and the cells were incubated and stained on ice in FBS (produced by Fluidigm) for 1 minute. After the cells were treated with a fixation/permeabilization buffer (produced by Thermo Fisher), the cells were mixed with the metal-labeled antibodies and incubated to label intracellular proteins. After the cells were cleaned, the cells were stained with 1 mL of 191/193Ir DNA intercalator (produced by Fluidigm) that was diluted at a ratio of 1:4000 (the intercalator was diluted with PBS containing 1.6% paraformaldehyde (produced by EMS)) and then stored at 4 °C. Before an assay, the cells were washed once with PBS containing 0.5% BSA and 0.02% NaN3, washed once with ddH₂O, and re-suspended and diluted to about 10⁶ cells/mL with ultrapure water (ddH₂O). Then, cell sample data was detected and collected using CyTOF2 (produced by Fluidigm) at an event rate of <400 events/sec.

According to the cellular immunophenotypic differences of 40 markers, a clustering analysis was performed through PhenoGraph clustering algorithm. ITNK cells derived from cord blood (hereinafter referred to as CB-ITNK), ITNK cells derived from peripheral blood (hereinafter referred to as PBMC-ITNK), and Mock-T cells were integrated and classified into 39 subgroups, as shown in FIGS. 12, 13, 14, 15, 16 and 17. As can be seen from FIG. 12, PX458-T cells and Mock-T cells were separated before and after stimulation by K562 cells, and there was almost no overlap, indicating that there was a significant difference between PX458-T cells and Mock-T cells; there was an obvious overlap between PX458-T cells before and after the stimulation by K562 cells, and more new subgroups were derived from activated PX458-T cells.

According to the results of a cell marker expression heterogeneity analysis through mass cytometry, the ITNK cells of the present application include a CD3-negative cell subgroup of NO. 33, CD4+ cell subgroups of Nos. 5 to 10, CD8+ cell subgroups of NOs. 20 to 22 and 26 to 28, and TCRγδ+ cell subgroups of NOs. 23 and 24, and all these ITNK cells express NK-associated markers such as CD56, NKp30, NKp44, NKp46, and CD11C; and compared with γδT cells, TCRγδ+ ITNK cells perform high expression of three markers NKp46, NKp30, and NKp44, that is, (NKp46^{high} NKp30^{high} NKp44^{high}) (as shown in FIGS. 13 and 14A). The ITNK cells of the present application are different from conventional NK cells in that the ITNK cells of the present application do not express CD127, CD16, NKG2A, or KIR2DL2 (see FIG. 14B). Moreover, the ITNK cells of the present application perform low expression of immunosuppression checkpoints PD-1, CTLA-4, and FOXP3 (as shown in FIG. 14C). Since the high expression of the immunosuppression checkpoints will induce immune cells to be in an immunosuppressive state such as low functionality and failure, it indicates that the ITNK cells of the present application have a strong immune effect and are not easily suppressed by an immunosuppressive microenvironment such as a tumor.

In addition, as for ITNK cells derived from cord blood, the histogram shown in FIG. 15 clearly shows the percentages of various ITNK cells in CD45+ hematopoietic cells and the dynamic transition from static ITNK cells to effector cells after stimulation. As shown in FIG. 16, the dynamic transition of ITNK cells derived from adult peripheral blood from a rest state to an effector state after stimulation is the same as the dynamic transition of the ITNK cells derived from cord blood. As shown in FIG. 17, the mass cytometry heatmap shows the dynamic changes of immunophenotyping of ITNK cells before and after activation and the increased expression of CD25 after activation. To sum up, ITNK cells, after activated, still maintain the expression of NK cell activation receptors and T cell markers.

### Example 4 Analysis of an RNA-Seq transcription profile of ITNK cells of the present application

To study the entire gene expression profile of ITNK cells, the inventors performed RNA sequencing and analysis on T cells derived from 4 cord blood samples and 3 adult peripheral blood samples, ITNK cells derived from 4 cord blood samples and 3 adult peripheral blood samples, and NK cells derived from 2 cord blood samples and 2 adult peripheral blood samples. The sorting operation was as follows: flow cytometry analysis or sorting was performed by flow cytometers Canto, FACS Fortessa (BD), FACSAriaII, etc. Cell surface receptors were labeled as follows: cells and antibodies were mixed in 50 µL of flow buffer (PBS solution containing 2% FBS) and incubated at 4°C for 30 minutes in the dark. Intracellular labeling: the cells were subjected to permeable treatment with Foxp3/transcription factor staining buffer (produced by eBioscience), and after the buffer was eluted, the cells were blocked with mouse serum or rabbit serum, incubated with antibodies at 4°C for 30 minutes in the dark, washed once with the flow buffer, and then re-suspended for subsequent flow cytometry analysis or sorting. A cell sorting strategy and the verification of sorting purity were shown in FIG. 18.

A principal component analysis (PCA) was performed for similarity evaluation on the RNA sequencing results of 18 samples. It was found that ITNK cells were different from T cells and NK cells according to a transcriptome analysis (as shown in FIG. 19). Compared with the T cell, the ITNK cell includes 776 genes increased (which is not shown by the data), which include NKspecific transcription factors (such as ID2, TBX21, NFIL3, and IRF8), NK cell activation and inhibition receptors/proteins (such as IL2RB, IFNG, PRF1, GZMB, TNFRSF4, NCR1, NCR2, and PLCG2), and histone genes (HIST1H1D, HIST1H2AC/D/E/G/M, and HIST2H2A3/4) (as shown in FIGS. 22 and 20). On the contrary, compared with the T cell, the ITNK cell includes 592 genes downregulated, such as TCF-1/TCF-7, LEF-1, IL-7R, MYC, PD-L1, and FOXP3 (as shown in Table 3). Compared with the NK cell, the ITNK cell includes 666 genes whose expression is increased (which is not shown by the data), most of which are enriched in T cell recognition and TCR signal transduction (CD3, CD4, CD8, and CD40LG) (FIG. 21). Interestingly, compared with the NK cell, the ITNK cell downregulates the expression of KIR genes KIR2DL1, KIR2DL3, KIR3DL1, and KIR3DL2 (as shown in Table 4), where KIR2DL1, KIR2DL3, KIR3DL1, and KIR3DL2 are NK cell inhibition receptors and mediate the inhibition function of immune cells, and their low expression indicates that the ITNK cell has resistance to the immunosuppressive microenvironment. The analysis results of whole transcriptome sequencing show that the expression of NK cell marker genes IL2RB, ID2, and NFIL3 is upregulated in the ITNK cell (shown in the right image of FIG. 22).

**Table 3 Expression of related genes of the ITNK cell relative to the NK cell**

| ENTREZ ID | Mark er | Basic value | log2 fold change | lfcSE | stat | P value | padj |
|---|---|---|---|---|---|---|---|
| 6932 | TCF7 | 6118.395 88 | -1.7050171 | 0.20634 066 | 8.26311 76 | 1.4192E -16 | 3.4959E -14 |
| 51176 | LEF1 | 4011.123 15 | -1.1451791 | 0.15917 866 | 7.19430 03 | 6.2782E -13 | 6.9436E -11 |
| 3575 | IL7R | 1810.031 57 | -3.9931208 | 0.40305 169 | 9.90721 77 | 3.8728E -23 | 6.2964E -20 |
| 4609 | MYC | 1160.228 97 | -2.6861929 | 0.28569 782 | 9.40221 67 | 5.3425E -21 | 4.1361E -18 |
| 50943 | FOXP 3 | 769.3022 46 | -2.0215289 | 0.58426 298 | 3.45996 4 | 0.00054 025 | 0.00415 092 |

**Table 4 Expression of related genes of the ITNK cell relative to the NK cell**

| ENTREZ ID | Marker | Basic value | log2 fold change | lfcSE | stat | P value | padj |
|---|---|---|---|---|---|---|---|
| 3811 | KIR3D L1 | 365.0991 98 | -7.1254794 | 0.75146 844 | 9.48207 4 | 2.4928E -21 | 3.971E-18 |
| 3804 | KIR2D L3 | 473.4124 88 | -4.8525337 | 0.89072 948 | 5.44781 98 | 5.0991E -08 | 2.9645E -06 |
| 3812 | KIR3D L2 | 373.9606 1 | -3.0917182 | 0.86143 947 | 3.58901 39 | 0.00033 193 | 0.00378 501 |
| 3802 | KIR2D L1 | 282.0004 46 | -4.7696308 | 0.99147 586 | 4.81063 73 | 1.5045E -06 | 5.0993E -05 |

### Example 5 Analysis of single-cell transcriptome sequencing of ITNK cells of the present application

Flow cytometry shows that CD8+CD3+NKp46+ ITNK and CD4+CD3+NKp30+ ITNK appear on day 5 after BCL11B knockout (as shown in FIG. 23). To further explain the cell fate transition during reprogramming of T cells into ITNK cells, the gene expression profiles of T cells and ITNK cells in CD3+ cord blood samples at different time points after BCL11B knockout were studied through micropore-based single-cell transcriptome sequencing (scRNA-seq).

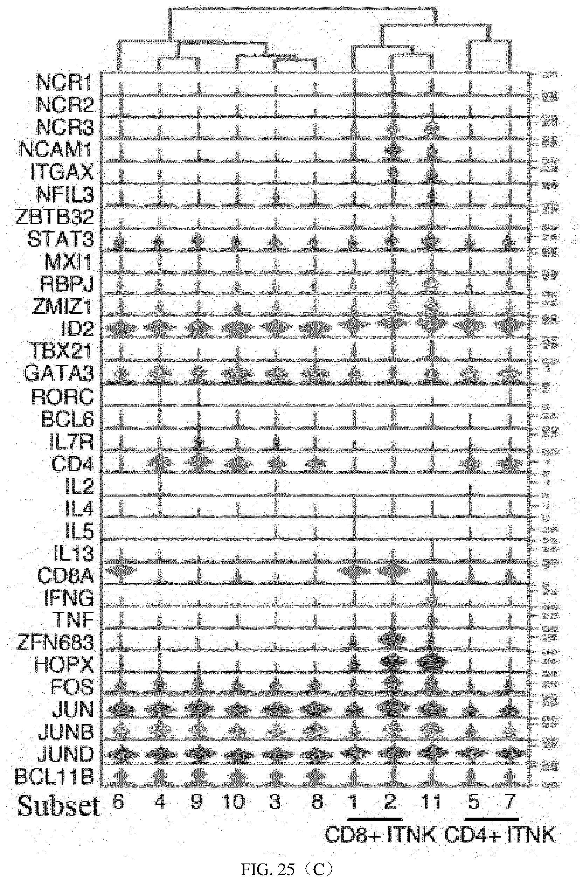

About 5000 cells were detected and analyzed in all experimental groups. Groups of cell samples at different time points were detected through scRNA-seq, an average of 2000-4000 genes were detected per cell, and a total of 20000 human genes were detected in all cells. In the t-distributed random neighbor-embedded (t-SNE) analysis of transcription profiles, the cells were projected to two dimensions, which provided the visual representation of the cell fate transition in the reprogramming process of ITNK cells. The results of the unbiased t-SEN analysis show that the cells from day 0 to day 20 after knockout can be clustered into 11 subgroups (as shown in FIG. 24). According to the expression of marker genes in T cells and NK cells, it is determined that ITNK cells are mainly concentrated in subgroup 6 (CD4+ ITNK), subgroup 1 (CD8+ ITNK), and subgroup 10 (γδTCR+ ITNK) (as shown in FIG. 24), and these subgroups completely coincide with BCL11B-deficient subgroups, which further indicates that ITNK cells are reprogrammed from T cells through BCL11B knockout. The KEGG enrichment analysis shows that ITNK cells specifically perform high expression of NK marker genes and associated genes (as shown in FIG. 25). As can be seen from FIGS. 24C and 25C, NOTCH1, NOTCH2, ZMIZ1 (NOTCH1 cofactor), and RBPJ (NOTCH downstream transcription factor) are upregulated in human ITNK cells, indicating that the NOTCH signaling pathway plays a role in the reprogramming of ITNK cells. FOS, JUN, and JUNB, three subunits of the AP-1 transcription factor, are expressed at a low level in the early stage of ITNK reprogramming and their expression is gradually upregulated in the late stage of ITNK reprogramming (as shown in FIG. 25C). It can be seen that a NOTCH signal and an AP-1 signal are upregulated after ITNK reprogramming. T cells and ITNK cells are closely aggregated in the t-SNE dot plots, indicating that the transition from T cells to NK cells is almost synchronous. The gradual transition from CD8+ T cells to CD8+ ITNK cells can be seen from the analysis of the trajectory of NK marker genes of CD8+ T (subgroup 5), early CD8+ ITNK (subgroup 0), and late CD8+ ITNK (subgroup 1) (as shown in FIG. 26A). Similarly, the analysis of the trajectory of NK marker genes of CD4+ T cells and ITNK cells also shows the gradual transition from T cells to ITNK cells (FIG. 26B). Consistent with the analysis results of dynamic flow cytometry of immunophenotyping of ITNK cells (FIG. 23), T cells begin to be reprogrammed into ITNK cells on day 5 after BCL11B knockout. It is found that the expression of transcription factor genes (TBX2, ID2, etc.) is significantly upregulated during the reprogramming of T cells into ITNK cells, which is further verified through immunoblotting (as shown in FIG. 27).

### Example 6 Ability of ITNK cells of the present application to recognize and kill MHCI-positive/negative tumor cells in vitro

To determine whether an NK-cell receptor (NCR) and a T-cell receptor (TCR) expressed by ITNK cells of the present application are functional, the ITNK cells were stimulated with an anti-NKp30 monoclonal antibody, an anti-NKp46 monoclonal antibody, and an anti-CD3/CD28 monoclonal antibody, separately. It is found that after stimulated with the anti-NKp30 antibody and the anti-NKp46 antibody, the ITNK cells secrete more interferons (IFNs) while T cells in the control group secrete the same IFNs (as shown in FIG. 28); and after stimulated with the anti-CD3/CD28 antibody, the ITNK cells secrete more interferons while the T cells in the control group secrete the same IFNs (as shown in FIG. 28), which indicates that the NCR and the TCR in the ITNK cells are functional.

Similar to NK cells, the ITNK cells of the present application can secrete a variety of cytokines including GM-CSF, IFN and TNF (as shown in FIG. 29), and can recognize and kill MHCInegative K562 cells (as shown in FIG. 30A). In addition, the ITNK cells can effectively kill Hela cells and A549 cells (as shown in FIG. 30B and 30C), both of which are ligands with the high expression of NK activation receptors and are MHCI-positive. As shown in Table 2, since the ITNK cells perform low expression of NK cell KIR receptors (KIR2DL1, KIR2DL3, KIR3DL1, and KIR3DL2) which mediate immunosuppression, the ITNK cells have a better tumor-killing effect than the NK cells. However, NALM-6 does not express an NCR ligand and highly expresses MHCI molecules, and the ITNK cells and the NK cells have no significant killing effect on NALM-6 (as shown in FIG. 30D). Then, the inventors stimulated ITNK cells, NK cells, and T cells with K562 cells, separately and found that the expression levels of phosphorylated Fyn, PLC-y2, Syk, Erk, and m-TOR in ITNK cells and NK cells were similar but higher than the expression levels in T cells (as shown in FIG. 31). These results indicate that ITNK cells have similar NK cell functions in terms of NCR activation, cytokine secretion, cytotoxicity, and signaling pathway.

### Example 7 Ability of ITNK cells of the present application to inhibit tumor growth in vivo

The inventors also evaluated whether the ITNK cells of the present application can inhibit the growth of xenograft tumors. Specifically, K562 cells labeled with luciferase were implanted into NSI mice to construct K562 tumor-bearing mouse models, and then ITNK cells, NK cells, or T cells were injected for a single time (FIG. 32A). The survival states of K562 cells in mice were detected at particular time points by living imaging equipment. Compared with the group injected with T cells (negative control) and the group injected with PBS (blank control), the experimental mice treated with ITNK cells and NK cells have significantly reduced K562 tumor loads after 28 days of injection (FIGS. 32B and 32C) and survive for a longer time (FIG. 32D). In addition, the inventors also transplanted Hela cells into NSI mice and treated Hela xenograft mice with ITNK cells, NK cells, and T cells, separately. The results show that the Hela tumor growth rate of tumor-bearing mice treated with ITNK cells is significantly slower than those of the group treated with NK cells, the group treated with T cells, and the group treated with PBS (FIG. 32E). It can be seen that the ITNK cells of the present application are effective killers of tumor cells *in vivo* and can prevent tumor growth.

### Example 8 Evaluation of the in vivo safety of ITNK cells of the present application

To verify the *in vivo* distribution and maintenance ability of the ITNK cells, the ITNK cells were transplanted into NSI-strain immunodeficient mice lacking T cells, B cells, and NK cells, and the percentages of ITNK cells in peripheral blood (PB), spleen (SP), bone marrow (BM), liver, and lung were measured on day 1, day 7, day 14, day 21, and day 180 after transplantation (FIGS. 33A and 33B). The proportion of ITNK cells peaks on day 21 after transplantation and then gradually decreases, and the ITNK cells cannot be detected after 6 months (FIG. 33B). As can be seen from FIG. 33B, the ITNK cells have a stronger *in vivo* maintenance ability than the T cells. The case where the ITNK cells attack a host or proliferate without restriction has not been observed.

To evaluate the possible off-target mutation induced by PX458-gBCL11B, T cells electroporated with PX458-gBCL11B were subjected to whole genome sequencing of high coverage. Compared with wild-type T cells, it is found from two independent experiments that there are very few off-target mutations caused by nuclease in the T cells edited by PX458-gBCL11B.

### Example 9 Construction of CAR ITNK cells of the present application

Although ITNK cells have TCR and NCR functions, the ITNK cells cannot recognize particular tumor antigens. For this purpose, the inventors transduced PB-CAR molecular vectors (the structure of a CAR molecule: an extracellular domain is the extracellular fragment of a receptor of an antigen such as CD19, GPC3, MUC1, or Mesothelin or the scFv sequence of the corresponding antibody, a transmembrane region is one or two of transmembrane regions of receptors CD28, NKG2D, NKp44, and NKp46, and an intracellular costimulatory domain is an intracellular costimulatory domain of CD28, TLR2, 2B4, DAP10, or DAP12, and CD3ζ ) and BCL11B knockout vectors PX458-gBCL11B into human T cells successively or simultaneously to obtain the ITNK cells expressing anti-CD19 chimeric antigen receptor (CAR) molecules. The expression of PI labels and GFP in the cells after transduction was detected through flow cytometry to determine the survival and transduction efficiency of the cells after transduction (as shown in FIG. 34 A) and the reprogramming into ITNK cells (as shown in FIG. 34B). Specifically, the percentage of CAR19-ITNK cells (19T2/ITNK as shown in FIG. 34) was detected on day 10 to day 14, where the CAR19-ITNK cells manifested as a GFP+CD4+NKp30+ subgroup, a CD4-CD8-γδITNK subgroup, and a GFP+CD8+NKp46+ subgroup.

### Example 10 Ability of CAR ITNK cells of the present application to kill CD19+ CML and BALL in vitro

To evaluate the anti-tumor effect of CAR19 (FMC63 scFv fragment-CD28 transmembrane region-CD28 and TLR2 intracellular domain-CD3ζ signal domain)-ITNK cells, human chronic myeloid leukemia cell line K562 cells (K562-CD19) expressing human CD19 and luciferase and B acute lymphocytic leukemia NALM-6 cells expressing luciferase were constructed. CAR19-ITNK cells, CAR19-T cells, NK cells, and T cells were mixed with the two leukemia cell lines at different E:T (effector cell : target cell) ratios for 24 h, respectively. Luciferase substrates were added and the killing situation of tumor cells was detected by a microplate reader.

The *in vitro* killing experiment on K562-CD19 cells shows that the CAR19-ITNK cells of the present application more effectively recognize and kill K562-CD19 cells than CAR19-T cells and ITNK cells (as shown in FIG. 35 A). However, when NALM-6 cells are eliminated, the CAR19-ITNK cells and the CAR19-T cells have similar killing abilities (as shown in FIG. 35B). This may be because NALM-6 is deficient in HLA-I-positive or NCR ligands. The ITNK cells have never lysed NALM-6 (as shown in FIG. 35B). These results indicate that the CAR signal and the NCR signal are compatible and can function together.

### Example 11 Ability of CAR19 ITNK cells of the present application to kill CD19+ CML in vivo

To detect the *in vivo* anti-tumor activity of CAR19-ITNK cells of the present application, the applicant injected the K562-CD19 cells constructed in Example 10 into NSI mice through veins (5×10⁵ cells per mouse), and then CAR19-ITNK cells, ITNK cells, CAR19-T cells, or T cells were injected (2.5×10⁵ cells per mouse), which were respectively referred to as the CAR19-ITNK group, the ITNK group, the CAR19-T group, or the T cell group. The experimental process is shown in FIG. 36A: on day 2, day 7, day 14, day 21, and day 28 after transplantation of K562-CD19, the leukemia mice were treated with luciferase substrates (which can interact with expressed luciferase, where luciferase-expressing cells can be detected by a living imager), the survival or killing situation of luciferase-labeled K562-CD19 cells in the leukemia mice was detected through a living imaging technique, and the distribution of luciferase-labeled cells was detected by the living imager, and the number of labeled cells was determined by a fluorescence intensity.

The experimental results show that mice in the CAR19-ITNK group have lighter tumor loads than the other groups as shown in FIGS. 36B and 36C. The experimental results also show that CAR19-ITNK cells more significantly inhibit NALM-6 growth than other cells such as CAR19-T cells and ITNK cells, as shown in FIGS. 36B and 36C. It is to be noted that although CAR19-ITNK cells and CAR19-T cells have similar abilities to kill NALM-6 *in vitro* (FIG. 35B), the CAR19-ITNK cells have better performance than CAR19-T cells in eliminating NALM-6 *in vivo.* To sum up, these results indicate that CARs enhance the cytotoxicity, especially *in vivo* cytotoxicity, of the ITNK cells to tumors.

### Example 12 Ability of anti-GPC3 CAR-ITNK cells of the present application to kill liver cancer cells

CAR-ITNK cells that recognize phosphatidylinositol GPC3 were constructed in the present application, where the structure of the CAR molecule includes an anti-GPC3 scFv extracellular fragment, an NKG2D transmembrane region, a 2B4 intracellular costimulatory domain, and CD3ζ. Four experimental groups, including CAR-ITNK cells, CAR-T cells, ITNK cells, and T cells, were set up in a 96-well plate, and three duplicate wells were set up for each group. Each well was added with 10000 tumor cells (GPC3-positive tumor cell lines Huh7-GL and HepG2-GL, where GL was a luciferase gene marker) as target cells. Effector cells were added into the plate at an E:T ratio of 4:1, 2:1, 1:1, 1:2, 1:4, separately. After the effector cells were incubated with the tumor cells for 24 hours, luciferase substrates were added and the killing ratio of tumor cells was detected by a quantitative spectrophotometer. It is found through the analysis of the experimental results that CAR-ITNK cells have a better tumor killing effect than ITNK cells, CAR-T cells, and T cells (which is not shown by data).

### Example 13 Ability of anti-TGFβ CAR-ITNK cells of the present application to proliferate and kill solid tumor cells

CAR-ITNK cells that recognize cytokine TGFβ were constructed in the present application, where the structure of the CAR molecule includes an anti-TGFβ scFv extracellular fragment, a CD28 intracellular costimulatory domain, a TLR2 intracellular costimulatory domain, and CD3ζ. Four experimental groups, including CAR-ITNK cells, CAR-ITNK+TGFβ, ITNK cells, and ITNK cells+TGFβ, were set up in a 96-well plate, and five duplicate wells were set up for each group, with 10⁵ cells per well. 6, 24, 48, 72, and 96 hours after TGFβ (3 ng/mL) was added, the absolute number of cells in each well was recorded through cell counting, and the secretion of related immune effector cytokines in different experimental groups was detected by ELISA. Through the comparison and data analysis of statistical results, it is found that TGFβ inhibits the proliferation of ITNK cells and the secretion of immune effector cytokines, while the anti-TGFβ CAR-ITNK cells exhibit enhanced cell proliferation and secretion of immune effector cytokines in the presence of TGFβ.

To evaluate the killing effect of the anti-TGFβ CAR-ITNK cells on tumor cells, four experimental groups, including CAR-ITNK cells, CAR-ITNK+TGFβ, ITNK cells, and ITNK cells+ TGFβ, were set up in a 24-well plate, and three duplicate wells were set up for each group. Each well had 2×10⁵ effector cells and was added with 10⁵ tumor cells (tumor cell line HepG2with a luciferase gene marker) as target cells. 24 hours after the TGFβ cytokine was added, luciferase substrates were added and the killing ratio of tumor cells was detected by a fluorometer. Through the analysis of the experimental results, it is found that TGFβ inhibits the tumor killing effect of ITNK cells, while the presence of TGFβ relatively enhances the killing effect of the anti-TGFβ CAR ITNK cells on tumor cells (which is not shown by data).

### Example 14 Ability of anti-Mesothelin CAR-ITNK cells of the present application to kill solid tumor cells

CAR-ITNK cells that recognize Mesothelin were constructed in the present application, where the structure of the CAR molecule includes an anti-Mesothelin scFv extracellular fragment, a CD28 transmembrane region, a DAP10/DAP12 sequence, and CD3ζ. Four experimental groups, including CAR-ITNK cells, CAR-T cells, ITNK cells, and T cells, were set up in a 96-well plate, and three duplicate wells were set up for each group. Each well was added with 10000 tumor cells (Mesothelin-positive tumor cell lines BGC-823-GL and MKN-28-GL, where GL was a luciferase gene marker) as target cells. Effector cells were added into the plate at an E:T ratio of 4:1, 2:1, 1:1, 1:2, 1:4, separately. After the effector cells were incubated with the tumor cells for 24 hours, luciferase substrates were added and the killing ratio of tumor cells was detected by a quantitative spectrophotometer. These experimental results are similar to the results in the preceding example and show that CAR-ITNK cells have a better tumor killing effect than ITNK cells, CAR-T cells, and T cells (which is not shown by data).

Tumor and virus recognition and killing activation pathways of the CAR ITNK cells of the present application do not interfere with each other and have a mutual synergistic effect. The CAR ITNK cells of the present application can not only activate and recognize tumor- or virus-associated antigens through CAR molecules but also recognize tumor- or virus-associated antigens through the pathway of the NK-cell receptor and the TCR in ITNK cells. The CAR ITNK cells not only have an efficient specific killing effect on particular tumors and viruses so as to rapidly control tumor progression and virus deterioration but also have broad-spectrum anti-tumor and anti-virus effects so as to prevent the escape and recurrence of tumors and viruses. The CAR-ITNK technology of the present application solves the problems in the existing art of tumor antigen escape, recurrence, and low efficiency in the CAR T and CAR NK treatment.

The applicant has stated that although the detailed method of the present application is described through the examples described above, the present application is not limited to the detailed method described above, which means that implementation of the present application does not necessarily depend on the detailed method described above. It should be apparent to those skilled in the art that any improvements made to the present application, equivalent replacements of raw materials of the product of the present application, additions of adjuvant ingredients to the product of the present application, and selections of specific manners, etc., all fall within the protection scope and the disclosure scope of the present application.

## Claims

1. An engineered immune killer cell prepared by transfecting a human T cell with a CAR molecule or a TCR molecule targeting a tumor-associated antigen or a virus-associated antigen along with or followed by reprogramming involving deletion or inhibition of a BCL11B gene.

2. The cell according to claim 1, wherein the immune killer cell expresses the CAR molecule or the TCR molecule targeting the tumor or the virus-associated antigen, retains a marker and a function of the human T cell from which the immune killer cell is derived, and has a marker and a function of NK cells.

3. The cell according to claim 1, wherein the human T cell is a mature human T cell or a cell population containing mature human T cells;
preferably, the mature human T cell or the cell population containing mature human T cells is derived from cord blood or peripheral blood of a human body;
preferably, the mature human T cell or the cell population containing mature human T cells is derived from a mature T cell or a cell population obtained through differentiation of pluripotent stem cells, embryonic stem cells, or cord blood stem cells.

4. The cell according to any one of claims 1 to 3, wherein the immune killer cell expresses functional TCR, CD3, and NKp30.

5. The cell according to any one of claims 1 to 4, wherein the immune killer cell expresses the following marker of NK cells: CD11c, NKG2D, and CD161;
preferably, the immune killer cell performs low expression or no expression of an immunosuppression checkpoint PD-1, CTLA-4, or FOXP3;
preferably, the immune killer cell performs low expression or no expression of an NK-associated marker CD127, CD16, KIRDL2, KIRDL3, NKG2A.

6. The new immune killer lymphocyte according to any one of claims 1 to 5, wherein the immune killer cell upregulates expression of NOTCH compared with the T cell from which the immune killer cell is derived.

7. The cell according to any one of claims 1 to 6, wherein the immune killer cell downregulates expression of transcription factors LEF1 and TCF7 and upregulates expression of NOTCH, AP1, ID2, TBX21, and NFIL3 compared with the T cell from which the immune killer cell is derived.

8. The cell according to any one of claims 1 to 7, wherein TCR-mediated signal transduction of the immune killer cell is enhanced;
preferably, compared with the T cell from which the immune killer cell is derived, the immune killer cell upregulates expression of genes CSF2, FOS, MAPK12, MAP3K8, IFNγ, NFKBIA, MAPK11, IL-10, and TEC which are associated with the TCR-mediated signal transduction;
preferably, compared with NK cells, the immune killer cell has enhanced T cell recognition and TCR signal transduction; preferably, the immune killer cell upregulates expression of CD3, CD4, CD8, and CD40LG.

9. The cell according to any one of claims 1 to 8, wherein compared with the T cell from which the immune killer cell is derived, the immune killer cell has enhanced NK killing toxicity-associated signal transduction;
preferably, compared with the T cell from which the immune killer cell is derived, the immune killer cell upregulates expression of genes PRF1, CSF2, ICAM1, CD244, PLCG2, IFNG, FCER1G, GZMB, NCR2, NCR1, KIR2DL4, and SYK which are associated with the NK killing toxicity-associated signal transduction.

10. The cell according to any one of claims 1 to 9, comprising CD8+NKp46+ NKp44+ NKp30+, CD4+NKp30+, and γδTCP+NKp46+ NKp44+NKp30+ T cell subgroups.

11. The cell according to any one of claims 1 to 10, wherein the human T cell is a mature human T cell, and reprogramming the mature human T cell comprises:
(1') activating the mature human T cell;
(2') performing BCL11B gene knockout on the activated mature human T cell obtained in step (1'); and
(3') culturing the cell obtained in step (2') in a T cell culture medium.

12. The cell according to claim 11, wherein in step (1'), the mature human T cell is activated using an anti-human CD3 antibody, an anti-human CD28 antibody, and an anti-human CD2 antibody; preferably, the T cell is activated through incubation of magnetic beads of the anti-human CD3 antibody, the anti-human CD28 antibody, and the anti-human CD2 antibody mixed with the mature human T cell at a ratio of 1:2.

13. The cell according to claim 11 or 12, wherein in step (2'), the BCL11B gene knockout is performed using CRISPR/CAS9 technology;
preferably, a target of the gene knockout is at a second exon of the BCL11B gene;
preferably, the target of the gene knockout is at a third exon of the BCL11B gene.

14. The cell according to any one of claims 11 to 13, wherein in step (3'), the T cell culture medium comprises IL-2; preferably, the cell obtained in step (2') is not co-cultured with OP9-DL1.

15. The cell according to any one of claims 1 to 14, wherein the CAR molecule comprises a signal peptide, an extracellular antigen recognition domain, a transmembrane region, and an intracellular costimulatory domain;
preferably, the CAR molecule comprises the signal peptide, the extracellular antigen recognition domain, the transmembrane region, and the intracellular costimulatory domain in sequence from an N-terminal to a C-terminal.

16. The cell according to any one of claims 1 to 14, wherein the tumor-associated antigen is a tumor surface antigen, a cytokine secreted by a tumor, a surface antigen of a cell associated with immunosuppression of a tumor microenvironment and a cytokine secreted by the cell, or a tumor-associated microbial antigen, preferably the tumor surface antigen, more preferably CD19, GPC3, Mesothelin, PSCA, or MUC1.

17. A method for preparing the cell according to any one of claims 1 to 16, comprising:
(1") activating a human T cell;
(2") transfecting the activated human T cell with a CAR molecule expressing a tumor-associated antigen or a tumor-specific TCR molecule along with or followed by performing BCL11B gene knockout; and
(3") culturing the cell obtained in step (2") in a T cell culture medium.

18. The method according to claim 17, wherein in step (1"), the human T cell is a mature human T cell or a cell population containing mature human T cells;
preferably, the mature human T cell or the cell population containing mature human T cells is derived from cord blood or peripheral blood of a human body;
preferably, the mature human T cell or the cell population containing mature human T cells is derived from a mature T cell or a cell population obtained through differentiation of pluripotent stem cells, embryonic stem cells, or cord blood stem cells.

19. The method according to claim 17 or 18, wherein in step (1"), the human T cell is activated using an anti-human CD3 antibody, an anti-human CD28 antibody, and an anti-human CD2 antibody;
preferably, the T cell is activated through incubation of magnetic beads of the anti-human CD3 antibody, the anti-human CD28 antibody, and the anti-human CD2 antibody mixed with the mature human T cell at a ratio of 1:2.

20. The method according to any one of claims 17 to 19, wherein in step (2"), the CAR molecule comprises a signal peptide, an extracellular antigen recognition domain, a transmembrane region, and an intracellular costimulatory domain; preferably, the CAR molecule comprises the signal peptide, the extracellular antigen recognition domain, the transmembrane region, and the intracellular costimulatory domain in sequence from an N-terminal to a C-terminal;
preferably, the antigen is the tumor-associated antigen and/or an antigen associated with a microorganism such as a virus or a bacterium;
preferably, the tumor-associated antigen is a tumor surface antigen, a cytokine secreted by a tumor, a surface antigen of a cell associated with immunosuppression of a tumor microenvironment and a cytokine secreted by the cell, or a tumor-associated microbial antigen, preferably the tumor surface antigen, more preferably CD19, GPC3, Mesothelin, PSCA, or MUC1.

21. The method according to any one of claims 17 to 20, wherein in step (2"), the BCL11B gene knockout is performed using CRISPR/CAS9 technology;
preferably, the gene knockout is performed at a second exon of a BCL11B gene;
preferably, the gene knockout is performed at a third exon of the BCL11B gene.

22. The method according to any one of claims 17 to 21, wherein in step (3"), the T cell culture medium comprises IL-2; preferably, the cell obtained in step (2") is not co-cultured with OP9-DL1.

23. A use of the cell according to any one of claims 1 to 16 for preparing a drug for treatment of a disease selected from the group consisting of a tumor, AIDS, and an infectious disease; preferably, the infectious disease is a viral infectious disease.
